# EUROPEAN PATENT APPLICATION

(11) **EP 3 124 589 A1**
(43) Date of publication of application: **01.02.2017**
(21) Application number: 15769155.1
(22) Date of filing: 18.02.2015
(51) Int. Cl.: C12M 1/00, C12M 3/00, C12N 5/00

(54) **CELL CULTURE METHOD AND CELL CULTURE SYSTEM**

(30) Priority: 28.03.2014 JP 2014068974
(71) Applicant: Toyo Seikan Group Holdings, Ltd., Shinagawa-ku Tokyo 141-8627 (JP)
(72) Inventor: HATA, Norihiko, Tokyo 141-8627 (JP)
(74) Representative: Liebetanz, Michael
(86) International application number: PCT/JP2015/000764
(87) International publication number: WO 2015/145954

(57) **Abstract**

When cells are cultured in transferring them in a plurality of culture containers in sequence, not only risk of contamination can be reduced, but also the number of cells remaining in the culture container can be reduced, and provision of a heating mechanism of a culture medium can be omitted. The plurality of culture containers 2a, 2b and 2c are each provided with one port for transferring contents with other containers, a cell suspension is accommodated within the first container 2a, a culture medium container 1 that supplies a culture medium and the culture containers are connected by means of a tube 3 to start cell culture, after completion of the culture in the first container, a cell suspension is transferred to the second container 2b connected to the first container, a culture medium is supplied from the culture medium container to the first container, and after the lapse of a prescribed period of time, the culture medium is supplied from the first container to the second container, cell culture is conducted in the second container, and if a subsequent culture container is present, transfer of the cell suspension to the subsequent culture container, supply of a culture medium to the culture container, supply of the culture medium from the culture container to the subsequent culture container and cell culture in the subsequent culture container are conducted repeatedly for the remaining culture containers.

## Description

### Technical Field

The present invention relates to a cell culture method and a cell culture system in which cells, tissues, microorganisms or the like are cultured in an artificial environment.

### Background Art

In recent years, in the fields of production of pharmaceuticals, gene therapy, regenerative medical treatment, immunotherapy, or the like, it is required to culture a large amount of cells, tissues, microorganism or the like efficiently in an artificial environment In such cell culture, with an increase in cell density in a culture medium, depletion of components required for proliferation and accumulation of metabolites of cells themselves occur, whereby proliferation speed is lowered and the cell density is saturated. Therefore, when culturing cells in a certain scale, culture is normally conducted by repeating subculture such that the cell density can be kept appropriately.

When such subculture is conducted, there are cases where cells are transferred from a culture container having a small capacity to a culture container having a larger capacity. For example, the following is conducted. Specifically, by using a cell culture well plate, cells are added to each well together with a culture medium to start culture such that a cell density suited to the initial stage of culture is attained. After sufficiently proliferating in the wells, the cells are transferred to a cell culture flask, and a culture medium is added and the cells are cultured in correspondence with the degree of cell proliferation. Further, when cells are proliferated to a certain amount, the cells are transferred to a bag having a further large capacity or the like, supplement of a culture medium and subculture are repeatedly conducted, whereby a large amount of cells are cultured (see Patent Document 1, paragraph [0027] or the like).

Further, a method is known in which, when cells that have been frozen-stored are used, after recovering them by thawing, culture is conducted for several days by using a well plate in order to recover the function inherent to the cells (hereinafter, this method is often referred to as "curing culture"). After recovering the function of cells to their original state, the cells are transferred to a flask and cultured (hereinafter, this method is often referred to as "expansion culture"), and thereafter, activation culture is conducted (paragraph [0057] of Patent Document 2, or the like, for example).

However, in such cell culture, when transferring cells from the well plate to the flask, it is required to repeat pipetting a number of times, and the cells are required to be transferred to a new culture container such as a flask or a bag whenever subculture is conducted. As a result, not only operation is complicated but also cells may be damaged. Further, a problem arises that there is a possibility that risk of contamination is high.

On the other hand, a cell culture apparatus or the like that can reduce the risk of contamination are described in Patent Document 3 and Patent Document 4.

That is, Patent Document 3 discloses an automatic cell culture apparatus that is provided with an air-tight cell culture apparatus placed in an incubator, a liquid feeding means to selectively pour the liquids from a culture medium container, a washing liquid container and a marrow liquid container through a liquid feeding pipe air-tightly connected to the culture apparatus, and a liquid discharging means to discharge the liquid in the culture apparatus through a liquid discharging pipe. Due to such configuration, stem cells adhering to the culture apparatus are selectively separated, the culture medium is poured into the culture apparatus to culture the stem cells, and then a culture medium for differentiation is poured into the culture apparatus to differentiate the cells to the desired tissue cells.

Further, Patent Document 4 discloses an automatic subculture apparatus that can subculture adherent cells in a closed system by using a plurality of culture containers. Specifically, this document discloses a technology in which, to the plurality of culture containers, a culture medium supply tank in which a culture medium is accommodated, a washing liquid supply tank in which a washing liquid is accommodated, a detaching liquid supply tank in which a detaching liquid is accommodated and a treatment container are connected, and a filter for preventing passage of cells is provided in the treatment container, and after cells are collected on the surface of the filter, a culture medium is supplied from the outside of the filter, whereby the cells are transferred by being pushed away by the culture medium.

### Related Art Documents

### Patent Documents

Patent Document 1: JP-A-2011-241159
Patent Document 2: JP-A-2013-215141
Patent Document 3: JP-A-2007-312668
Patent Document 4: JP-A-2005-198626

### Disclosure of the Invention

### Problem to be Solved by the Invention

However, it cannot be said that these cell culture apparatuses or the like are optimum when culturing a large amount of cells by using a plurality of cell culture containers.

That is, according to the cell culture apparatus of Patent Document 3, it is possible to culture stem cells in a single culture container to differentiate them into desired tissue cells. However, this culture apparatus cannot be preferably utilized in subculture or the like that becomes necessary when a large amount of cells are cultured.

Further, in the automatic subculture apparatus of Patent Document 4, subculture of cells is conducted by using a plurality of culture containers. In this apparatus, after collecting cells that have been cultured in a single culture container on the surface of a filter, a culture medium is supplied from the outside of the filter to allow the cells to be transferred to three culture containers to re-culture the cells. After collecting the re-cultured cells to the surface of the filter, a culture medium is supplied from the outside of the filter to collect part or all of cells. In this culture apparatus, although part of the re-cultured cells can be subcultured, all of the re-cultured cells cannot be subcultured. Therefore, it cannot be said that this apparatus is suited to culture of a large amount of cells. In addition, there is a problem that the procedure is complicated. Further, in this technology, since cells are transferred to or collected from a culture container after flowing the cells together with a culture medium or a washing liquid and blocking them by a filter, the cells may be damaged.

When conducting cell culture by using a plurality of culture containers, conventionally, a problem occurs that cells tend to remain in culture containers when transferring cells. If cells remain in a culture container, it is impossible to utilize all of the cells, and as a result, part of the cells has to be disposed. This causes lowering in proliferation efficiency of cells, posing a serious problem.

Further, as for a culture medium used in cell culture, the quality thereof is deteriorated unless being stored at low temperatures, and hence, a culture medium is stored by cooling until it is used. However, since cell culture is conducted at around 37°C, if the cooled culture medium is supplied as it is to a culture container in which cells and a culture medium are sealed, a problem arises that proliferation efficiency of cells is lowered.

Conventionally, such problems were solved by supplying a culture medium to the culture container after heating it by a heating mechanism such as a heater, as stated in paragraph 0018 of Patent Document 3, for example.

However, provision of a heating mechanism in a cell culture system makes configuration complicated and large-sized. Therefore, it is desired that no heating mechanism be provided.

The present invention has been made in view of the above-mentioned circumstances, and is aimed at providing a cell culture method and a cell culture system that are capable of reducing the risk of contamination when cell culture is conducted by using a plurality of culture containers and are capable of reducing the number of cells remaining in a culture container when transferring cells, and are capable of omitting the use of a heating mechanism for a culture medium.

### Means for Solving the Problem

The cell culture method according to the present invention is a cell culture method in which cells are cultured by using a plurality of culture containers, wherein
each of the plurality of culture containers is provided with one port for transferring contents with other containers,
a cell suspension containing cells and a culture medium is accommodated within a first culture container among the plurality of culture containers,
a culture medium supply container that supplies a culture medium to the plurality of culture containers and the first culture container are connected by means of a tube, and other culture containers among the plurality of culture containers are connected in sequence to the first culture container by means of a tube,
cell culture is started in the first culture container,
after completion of the culture in the first culture container, a cell suspension that contains cultured cells is transferred from the first culture container to a second culture container connected to the first culture container, and subsequently, a culture medium is supplied from the culture medium supply container to the first culture container, and after the lapse of a prescribed period of time, the culture medium is supplied from the first culture container to the second culture container to conduct cell culture in the second culture container, and
after completion of the culture in the second culture container, if there is a subsequent culture container connected to the culture container in which the culture has been completed, transfer of the cell suspension to the subsequent culture container, supply of a culture medium to the culture container in which the culture has been completed, supply of the culture medium from the culture container in which the culture has been completed to the subsequent culture container, and culture of cells in the subsequent culture container are conducted repeatedly until there is no subsequent culture container.

The cell culture system according to the present invention is a cell culture system that is provided with a plurality of culture containers for culturing floating cells, comprising:
a culture medium supply container that supplies a culture medium to the plurality of culture containers, and
as the plurality of culture containers,
a curing culture container used for cell culture for recovering functions of damaged cells,
an activation culture container used for cell culture for activating cells, and
one or two or more amplification culture containers used for cell culture for proliferating cells, wherein
each of the curing culture container, the activation culture container and the amplification culture container is provided with one port for transferring contents with other containers, and
the culture medium supply container, the curing culture container, the activation culture container and the amplification culture container are connected in this sequence by means of a tube.

Further, the cell culture system according to the present invention is a cell culture system that is provided with a plurality of culture containers for culturing adherent cells, comprising:
a culture medium supply container that supplies a culture medium to the plurality of culture containers, and
culture containers differing in culture area as the plurality of culture containers, wherein
each of the plurality of culture containers is provided with one port for transferring contents with other containers, and
the plurality of culture containers are connected, in the ascending order from the smallest to the largest in terms of culture area, by means of a tube to the culture medium supply container.

### Advantageous Effects of the Invention

According to the present invention, when cell culture is conducted by using a plurality of culture containers, it is possible to reduce the risk of contamination, and when transferring cells, it is possible to reduce the number of cells remaining in a culture container, and further, it is possible to omit provision of a heating mechanism of a culture medium.

### Brief Description of the Drawings

Fig. 1 is an explanatory view showing an outline of the cell culture system according to the first embodiment of the present invention;
Fig. 2 is an explanatory view showing an outline of stirring means in the cell culture system according to the first embodiment of the present invention;
Fig. 3 is a perspective view showing a primary culture step in the cell culture method according to the first embodiment of the present invention;
Fig. 4 is a perspective view showing a cell transfer step in the cell culture method according to the first embodiment of the present invention;
Fig. 5 is an explanatory view showing an outline of the cell culture system according to the second embodiment of the present invention;
Fig. 6 is a perspective view showing an outline of a curing container and a retaining tool in the cell culture system according to the second embodiment of the present invention;
Fig. 7 is an explanatory view showing an outline of the cell culture system according to the third embodiment of the present invention; and
Fig. 8 is an explanatory view showing an outline of the cell culture system according to the fourth embodiment of the present invention.

### Mode for Carrying out the Invention

Hereinbelow, preferred embodiments of the cell culture method and the cell culture system according to the embodiment of the present invention will be explained with reference to the drawings.

### (First embodiment)

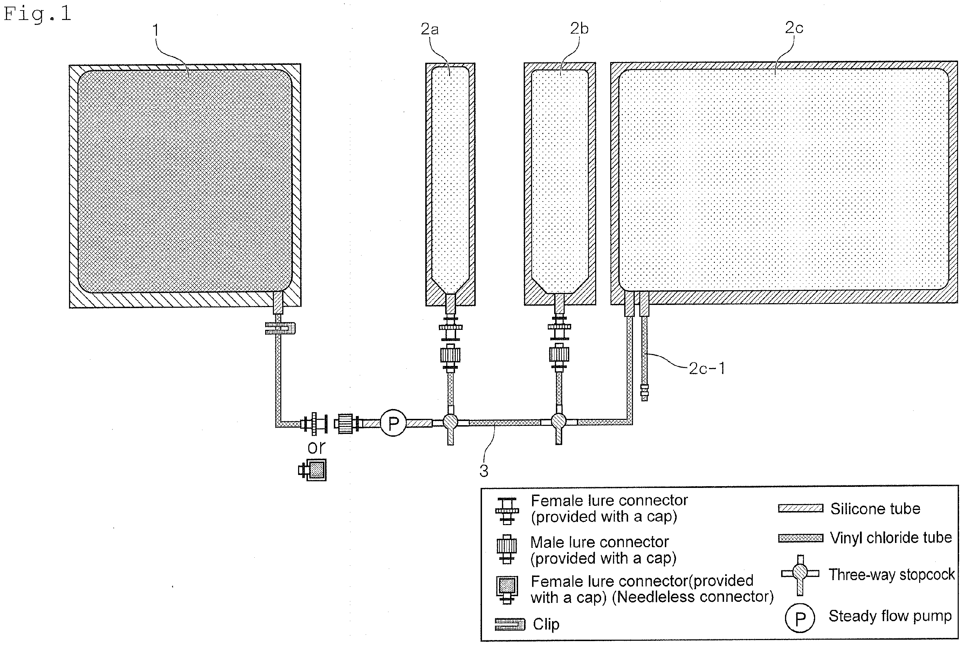

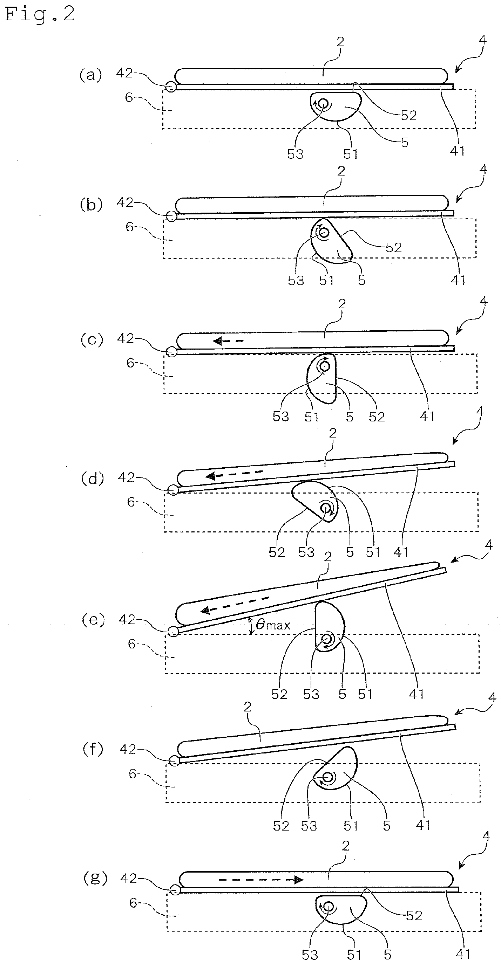

First, the cell culture method and the cell culture system according to the first embodiment of the present invention will be explained with reference to Fig. 1 and Fig. 2.

### [Cell culture system]

As shown in Fig. 1, the cell culture system according to the present embodiment is provided with a culture medium supply container 1 (hereinafter, often referred to as a culture medium container 1), a plurality of culture containers 2 (a primary culture container 2a, a secondary culture container 2b and a tertiary culture container 2c), and a transfer tube 3 (hereinafter, often simply referred to as a tube (conduit)).

Each of the plurality of culture containers 2 is provided with a port for transferring contents with other containers. The culture medium container 1 and the plurality of culture containers 2 are connected by a tube.

By the cell culture system according to the present embodiment, since subculture of cells can be conducted in a closed system by using these plurality of culture containers 2, risk of contamination can be reduced.

When culture is completed in a certain culture container 2, and culture is then conducted in a subsequent culture container 2 by using the cell culture system according to the present embodiment, before pouring a culture medium to these subsequent culture container 2, by pouring a culture medium to the culture container 2 in which the culture has already been completed and waiting for a predetermined period of time, and then transferring the culture medium to the culture container 2 in which the culture is subsequently conducted, the number of cells remaining in the culture container 2 in which the culture has been completed can be reduced, and the temperature of the culture medium to be transferred to the culture container 2 in which culture is subsequently conducted can be increased. Accordingly, proliferation efficiency of cells can be further improved.

These advantageous effects can be similarly obtained in the following embodiments.

### [Culture medium supply container]

The culture medium container 1 is a container for supplying a culture medium for culturing cells to a culture container, and stores a culture medium supplied to the plurality of culture containers 2. The culture medium container 1 may be formed of a plurality of containers in order to supply the same or different culture media.

It is preferred that the culture medium container 1 have gas barrier properties against oxygen and carbon dioxide in order to prevent pH of the culture medium stored from being changed largely during culture. The reason therefor is that, in order to prevent carbon dioxide having a high concentration contained in a culture medium from escaping to the air to lower the carbon dioxide concentration in the culture medium, leading to an increase in pH, it is desired that leakage of carbon dioxide from the inside of the culture medium container 1 to the outside be suppressed as much as possible. Further, it is desirable to prevent oxidation of a culture medium.

The culture medium container 1 is provided with one port. To this port, a transfer tube 3 is connected, and the culture medium container 1 is connected to a plurality of culture containers 2 through this transfer tube 3. From this culture medium container 1, a culture medium can be supplied to the culture container 2 by means of a pump in corresponding with proliferation or the like of cultured cells.

The culture medium container 1 can be used as a waste liquid collection container (hereinafter, often referred to as a waste liquid container) when it becomes empty after completion of culture. Then, by flowing a supernatant of a cell suspension from the culture container 2 to the culture medium container 1 as the waste liquid container, the cell suspension in the culture container 2 can be concentrated. Further, it is possible that, by connecting two or more culture medium containers 1 and using one as the waste liquid container and another as the cell collection container, a concentrated cell suspension is flown from the culture container 2 to the culture medium container 1, whereby cells are collected.

### [Culture container]

The culture container 2 is a container for proliferating cells by subculture or the like, and is composed of a plurality of containers. In the example shown in Fig. 1, as the culture container 2, a primary culture container 2a, a secondary culture container 2b and a tertiary culture container 2c are provided, and it is supposed that cell culture be conducted in this sequence. The number of the culture container 2 is not limited to three. It may be two, or may be four, five or more.

As for the capacity and the culture area of the culture container 2, it is preferred that those of containers being connected later be larger in order to conduct subculture or the like efficiently. For example, as a secondary culture container 2b, it is preferable to use one having a capacity twice or more as large as that of the primary culture container 2a and is preferable to use one having a culture area 1.5 times or more as large as that of the primary culture container 2a. Further, as the tertiary culture container 2c, it is preferable to use one having a capacity about 10 times as large as that of the secondary culture container 2b, and is preferable to use one having a culture area 6 times or more as large as that of the secondary culture container 2b. The ratios of the capacity and the culture area of the primary culture container 2a, the secondary culture container 2b and the tertiary culture container 2c are not. limited to those mentioned above.

In the cell culture system according to the present embodiment, after conducting cell culture in the primary culture container 2a, cells that have been cultured are transferred from the primary culture container 2a to the secondary culture container 2b. Subsequently, the culture medium is added from the culture medium container 1 to the primary culture container 2a. Then, in a state where the culture medium is stored in the primary culture container 2a, by waiting for a prescribed period of time, the temperature of the culture medium is increased. This waiting time varies depending on the amount and temperature of the culture medium, but is about 5 minutes to 10 minutes, for example. Subsequently, the culture medium is transferred from the primary culture container 2a to the secondary culture container 2b.

Further, when transferring the culture medium from the culture medium container 1 to the primary culture container 2a, it is preferable to allow the transfer speed to be slower than normal transfer speed. For example, it is preferable that this transfer speed be about 5% to 50% of the normal transfer speed. More specifically, the transfer speed is preferably 5 mL/min to 50 mL/min, and more preferably from 10 mL/min to 30 mL/min, for example. By allowing the transfer speed of the culture medium to be slower than normal transfer speed, prior to transfer to the culture container 2 in which subsequent culture is conducted (secondary culture container 2b), it is possible to increase the temperature of the culture medium.

Further, since the culture medium is transferred to the secondary culture container 2b after being transferred to the primary culture container 2a, it is possible to transfer cells remaining in the primary culture container 2a to the secondary culture container 2b together with the culture medium. Accordingly, the number of cells remaining in the culture container 2 in which the culture has been completed (primary culture container 2a) can be reduced.

After cell culture is conducted in the secondary culture container 2b, similarly, cultured cells are transferred from the secondary culture container 2b to the tertiary culture container 2c, and subsequently, the culture medium is added from the culture medium container 1 to the primary culture container 2a. Then, in a state where the culture medium is stored in the primary culture container 2a, by waiting for a prescribed period of time, the temperature of the culture medium is allowed to be increased. Subsequently, the culture medium is transferred from the primary culture container 2a to the secondary culture container 2b. Then, in a state where the culture medium is stored in the secondary culture container 2b, by waiting for a prescribed period of time, the temperature of the culture medium is allowed to be increased. Subsequently, the culture medium is transferred from the secondary culture container 2b to the tertiary culture container 2c.

At this time, since the culture medium is transferred to the tertiary culture container 2c after being transferred to the primary culture container 2a and the secondary culture container 2b, the cells remaining in the primary culture container 2a and the secondary culture container 2b can be transferred to the tertiary culture container 2c together with the culture medium.

Meanwhile, after cell culture is conducted in the secondary culture container 2b, after adding the culture medium only to the primary culture container 2a to increase the temperature thereof, the culture medium may be transferred to the tertiary culture container 2c. Alternatively, after adding the culture medium only to the secondary culture container 2b to increase the temperature thereof, the culture medium may be transferred to the tertiary culture container 2c. Further, after adding the culture medium to both the primary culture container 2a and the secondary culture container 2b to increase the temperature thereof, the culture medium may be transferred to the tertiary culture container 2c.

As mentioned above, by the cell culture system according to this embodiment, it is possible to significantly reduce the number of cells remaining in the culture container, and is possible to transfer the culture medium after increasing the temperature thereof, whereby proliferation efficiency of cells can be further improved.

These culture containers 2 are obtained by forming a soft packaging material into a bag-like shape, and part or all thereof has transparency such that contents can be confirmed visually.

Further, the culture container 2 is required to have permeability to gas (permeability to oxygen and carbon dioxide) required for cell culture. It is preferable to use the container under cell culture conditions of 37°C and 5% carbon dioxide concentration. Further, in order to realize a high cell proliferation efficiency, it is preferred that the culture container 2 have low cytotoxicity, low elution properties, and suitability to radiation sterilization.

As the material of the culture container 2 that satisfies such conditions, a polyethylene-based resin is preferable. As the polyethylene-based resin, polyethylene, a copolymer of ethylene and α-olefin, a copolymer of ethylene and vinyl acetate, an ionomer obtained by using a copolymer of ethylene and acrylic acid or methacrylic acid and a metal ion, or the like can be given. Further, polyolefin, a styrene-based elastomer, a polyester-based thermoplastic elastomer, a silicone-based thermoplastic elastomer, a silicone resin or the like can also be used.

Although the shape of the culture container 2 is not particularly restricted, it can be rectangular as shown in Fig. 1. The culture container 2 is provided with one port for transferring contents with other containers. To this port for transferring contents, the transfer tube 3 is connected. Through this transfer tube 3, injection of the culture medium from the culture medium container 1 to the culture container 2 and transfer of the culture medium or the cell suspension from the culture container 2 to the subsequent culture container 2 are conducted.

As for this transfer port, in order to allow contents to be naturally flown to the subsequent culture container 2 by inclining the culture container 2, it is desired that the port be arranged such that it is positioned at a lower side when the culture container 2 is inclined.

A culture container 2 in which cell culture is conducted finally (normally, a culture container 2 that is connected finally; a tertiary culture container 2c in Fig. 1) is provided with a port for sampling. A sampling tube 2c-1 is connected to this port. The sampling tube 2c-1 is used in order to take part of cultured cells from the culture container 2 with an aim of confirming the state of cultured cells, or the like. In the front end part of a free end of the sampling tube 2c-1 that is opposite to the port of the culture container 2, a two-stage seal is provided. By stroking the sampling tube 2c-1, the cell suspension can be filled in the sampling tube 2c-1 from the culture container 2. By cutting the sampling tube 2c-1 after heat sealing, cells can be sampled in a state that the cell culture system is kept in a closed state.

It is also preferred that a port for sampling be provided in an intermediate culture container 2 (the secondary culture container 2b in Fig. 1) and that a sampling tube be connected to this port, thereby to enable sampling of cells.

The four sides of the culture container 2 are sealed by heat sealing. The shape of the accommodation part of the culture container 2 may be rectangular. Alternatively, as shown in Fig. 1, it is preferred that the culture container 2 be configured to be gradually narrower towards the port. The culture container 2 may be an integrally-molded bag obtained by blow molding.

### [Transfer tube]

The transfer tube 3 connects the culture medium container 1 and the plurality of culture containers 2, whereby injection of the culture medium from the culture medium container 1 to the culture container 2 and transfer of the culture medium or the cell suspension between a plurality of culture containers 2 become possible.

In the flow passage of the transfer tube 3, a passage switching means and a pump are appropriately arranged, and a liquid flow passage is controlled by the passage switching means. The passage switching means is not particularly restricted, and a pinch valve or a stopcock, a three-way stopcock or the like can be used. In Fig. 1, the culture medium container 1 is connected with a plurality of culture containers 2 by means of three-way stopcocks, and transfer of the culture medium from the culture medium container 1 to the culture container 2 is conducted by means of a pump. Further, in this figure, connection of the culture containers 2 (a primary culture container 2a, a secondary culture container 2b) with the transfer tube 3 or connection of the transfer tubes 3 is conducted by means of a lure connector. However, connection may be conducted by other methods.

The material of the transfer tube 3 may be appropriately selected according to the environment of use. It is desirable to use one having excellent gas permeability for oxygen and carbon dioxide. For example, silicone rubber, soft vinyl chloride resins, polybutadiene resins, ethylene-vinyl acetate copolymers, chlorinated polyethylene resins, polyurethane-based thermoplastic elastomers, polyester-based thermoplastic elastomers, silicone-based thermoplastic elastomers, styrene-based elastomers or the like can be used. As the styrene-based elastomer, SBS (styrene-butadiene-styrene), SIS (styrene-isoprene-styrene), SEBS (styrene-ethylene-butylene-styrene), SEPS (styrene-ethylene-propylene-styrene) or the like can be used.

In Fig. 1, a silicone tube is used in a part of the transfer tube 3 at which a pump is attached. A soft vinyl chloride resin tubes are used in other parts.

### [Stirring means]

When cell culture is conducted by using the culture container 2, in order to promote proliferation of cells by keeping a good culture environment by allowing distribution of cells and concentration of components of the culture medium in the liquid contents in the culture container 2 to become uniform, it is preferable to provide stirring means that stirs liquid contents of the culture container 2 at the time of starting culture, at the time of supplying the culture medium or the like. The stirring means can be used for the purpose of stirring liquid contents of a culture container in the following embodiments, or for other purposes.

At this time, by the stirring means, by repeating an upward movement step in which the culture container 2 is moved upward to a position higher than the initial position and a downward movement step in which the culture container 2 is returned to the initial position and vibration is applied to the culture container 2 that has been returned to the initial position, liquid contents in the culture container 2 can be stirred.

When repeating the upward movement step and the downward movement step, in order to allow stirring of the liquid contents in the culture container 2 to be conducted without fail, according to need, the upward movement step is conducted at an interval (i.e. after the lapse of a prescribed period of time) after the completion of the downward movement step.

Due to such a configuration, in addition to stirring of the liquid contents by the upward and downward movements of the culture container 2, due to the vibration applied to the culture container 2 in the downward movement step, cells are diffused in the liquid contents as if they fly up. As a result, the liquid contents in the culture container 2 are efficiently stirred in the upward and downward directions, and while reducing the shear stress when cells roll down on the culture surface of the culture container 2 and minimizing damage on cells, distribution of cells and concentration of components of the culture medium in the liquid contents in the culture container 2 are allowed to be uniform, whereby a good culture environment can be maintained. In addition, adhesion of cells to the inner wall of the container or excessive agglomeration of cells can be suppressed, whereby proliferation of cells can be promoted. Further, when cells to be cultured are adherent cells, by vibration applied to the culture container 2 in the downward movement step, it is possible to promote detachment of cells from the culture surface of the culture container 2.

In order to stir the liquid contents in the culture container 2 as mentioned above, as shown in Fig. 2, for example, it is preferable to use a device comprising: a holding part 4 having a flat plate-shaped holding plate 41 in which a turning shaft 42 that is rotatably provided on one end thereof is pivotably supported on a base 6, and a cam mechanism in which the holding plate 41 is used as a follower, wherein the culture container 2 is held in the holding part 4, and the holding plate 41 is turned repeatedly upward and downward around the turning shaft 42 by the cam mechanism, and vibration generated when the holding plate 41 is turned downward and contacts with the base 6 is applied to the culture container 2, whereby the liquid contents in the culture container 2 are stirred. The plurality of culture containers 2 may be held separately by different holding plates 41. Alternatively, all of these culture containers 2 may be held by a single holding plate 41.

In such a device, in the upward movement step, the culture container 2 held in the holding part 4 starts to incline while moving upward from the initial position as the holding plate 41 is turned upward by the cam mechanism. As a result, while the culture container 2 that is formed of a flexible material is flexuously deformed, the liquid contents flow to the turning shaft 42 such that it is gathered on one side (see Figs. 2(a) to (e)). In the downward movement step, the holding plate 41, after reaching the maximum inclination angle θₘₐₓ, is turned downward, and as a result, the culture container 2 is returned to the initial position (see Figs. 2(f) and (g)). At this time, swing back occurs in the liquid contents in the culture container 2, and at the same time, the cells are diffused as if they fly in the liquid contents by vibration generated by impact occurred when the holding plate 41 contacts with the base 6, whereby the liquid contents in the culture container 2 are stirred.

When the liquid contents in the culture container 2 are stirred in this way, in order to further reduce the damage on the cells to allow the liquid contents in the culture container 2 to be stirred more efficiently, it is preferred that the holding plate 41 that is supported horizontally on the base 6 be turned upward slowly and after the holding plate 41 reaches the maximum inclination angle θₘₐₓ, the holding plate 41 be turned moved downward by falling, so that vibration is applied to the culture container 2 by impact that occurs when the falling holding plate 41 is received on the base 6. Although not particularly shown, when the vibration generated by impact occurred when the holding plate 41 contacts with the base 6 is applied to the culture container 2, it is possible to provide a convex part that contacts with the holding plate 41 in the base 6 at a position that is preferable for applying vibration to the culture container 2 in respect of efficient stirring of the liquid contents in the culture container 2.

Further, the holding plate 41 can be turned downward by falling, as follows, for example. A cam mechanism is constituted by a plate cam 5 having a first cam surface 51 having a circular contour and a second cam surface 52 having a linear contour, the curvatures of the cam surfaces 51 and 52, the position of the driving shaft 53 which drives the plate cam 5 and the rotational speed of the driving shaft 53 are appropriately set, and the holding plate 41 is turned upward by the first cam surface 51 and the plate cam 5 is moved away from the holding plate 41 when the plate cam 5 is switched to the second cam surface 52 (see Fig. 2(f)), whereby the holding plate 41 can be turned downward by falling.

Further, by allowing the plate cam 5 to be in contact with the holding plate 41 when switching to the second cam surface 52 and by appropriately setting the curvatures of the cam surfaces 51 and 52 and the position and the rotational speed of the driving shaft 53, it is possible to stir the liquid contents in the culture container 2 slowly by swinging the culture container 2 without applying impact to the culture container 2.

As mentioned above, the shape of the culture container 2 is normally rectangular, and the shape of the holding plate 41 is designed in accordance with the shape of the culture container 2. When the holding plate 41 is formed to be rectangular, the one end at which the turning shaft 42 is provided may be on the shorter side or may be on the longer side.

Such stirring means for the liquid contents in the culture container 2 can be used as cell detaching means when floating cells are detached from a culture surface after being activated or, as mentioned above, when adherent cells are detached from the culture surface.

Although not shown, it is preferred that the cell culture system be provided with an observation apparatus for observing the state of the cells or the culture medium in each culture container 2. As examples of observation apparatus, one formed of a CCD camera, an objective lens, an illumination, and information processing devices or the like can be used, for example. In this case, light is irradiated from above of the culture container 2 that is horizontally placed on a mounting table having an observation hole, and cells or the like in the culture container 2 can be automatically photographed at a prescribed timing by means of a CDD camera and an objective lens from below the observation hole. Then, the resulting image data is sent to an information processing device, and the image data is analyzed in the information processing device, whereby the number or density of cells in the culture container 2 can be calculated. The same can be applied to the following embodiments.

### [Cell culture method]

Subsequently, the cell culture method according to the present embodiment will be explained in detail with reference to Figs. 1 to 4.

The cell culture method according to the present embodiment is a cell culture method in which cells are cultured by using a plurality of culture containers, wherein
each of the plurality of culture containers is provided with one port for transferring contents with other containers,
a cell suspension containing cells and a culture medium is accommodated within a first culture container among the plurality of culture containers,
a culture medium supply container that supplies a culture medium to the plurality of culture containers and the first culture container are connected by means of a tube, and other culture containers among the plurality of culture containers are connected in sequence to the first culture container by means of a tube,
cell culture is started in the first culture container,
after completion of the culture in the first culture container, a cell suspension that contains cultured cells is transferred from the first culture container to a second culture container connected to the first culture container, and subsequently, a culture medium is supplied from the culture medium supply container to the first culture container, and after the lapse of a predetermined period of time, the culture medium is supplied from the first culture container to the second culture container to conduct cell culture in the second culture container, and
after completion of the culture in the second culture container, if there is a subsequent culture container connected to the culture container in which the culture has been completed, transfer of the cell suspension to the subsequent culture container, supply of a culture medium to the culture container in which the culture has been completed, supply of the culture medium from the culture container in which the culture has been completed to the subsequent culture container, and culture of cells in the subsequent culture container are conducted repeatedly until there is no subsequent culture container.

Specifically, the cell culture method according to the present embodiment comprises (1) container connection step; (2) primary culture step; (3) cell transfer step 1; (4) culture medium addition step 1; (5) secondary culture step; (6) cell transfer step 2; (7) culture medium addition step 2; (8) tertiary culture step; (9) sampling step; and (10) concentration step.

When the number of the culture container 2 in the cell culture system is two, the steps (6) to (8) are omitted, and a sampling tube is provided in the secondary culture container 2b to conduct steps (9) to (10). When the number of the culture container 2 in the cell culture system is four or more, the steps (6) to (8) are repeatedly conducted for the four or more containers. Further, the steps (9) and (10) can be omitted.

### (1) Container connection step

First, the culture medium container 1 and the plurality of culture containers 2 used in the cell culture method according to the present embodiment are connected as shown in Fig. 1, whereby a closed cell culture system is configured.

That is, the culture medium container 1, the primary culture container 2a, the secondary culture container 2b and the tertiary culture container 2c are connected in this sequence by means of the transfer tube 3.

At this time, connection is conducted in a state that a cell suspension containing cells to be cultured and a culture medium is accommodated within the primary culture container 2a. Further, connection is conducted in a state that the culture medium container 1 is filled with a culture medium to be supplied to the culture container 2. The secondary culture container 2b and the tertiary culture container 2c are connected in a state they are empty. On the transfer tube 3 between the culture medium container 1 and the culture container 2, a pump is provided. By this pump, transfer or the like of a culture medium is conducted. On the transfer tube 3, three-way stopcocks are appropriately provided, and the liquid flow passage can be controlled.

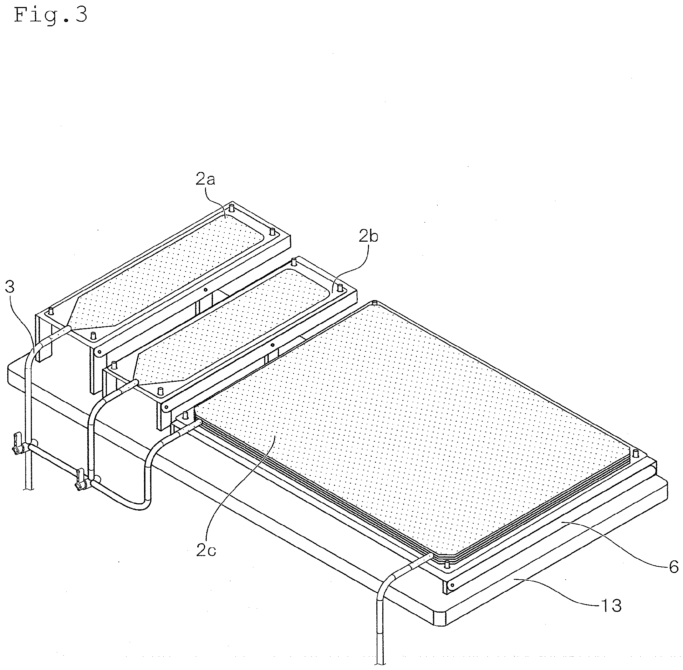

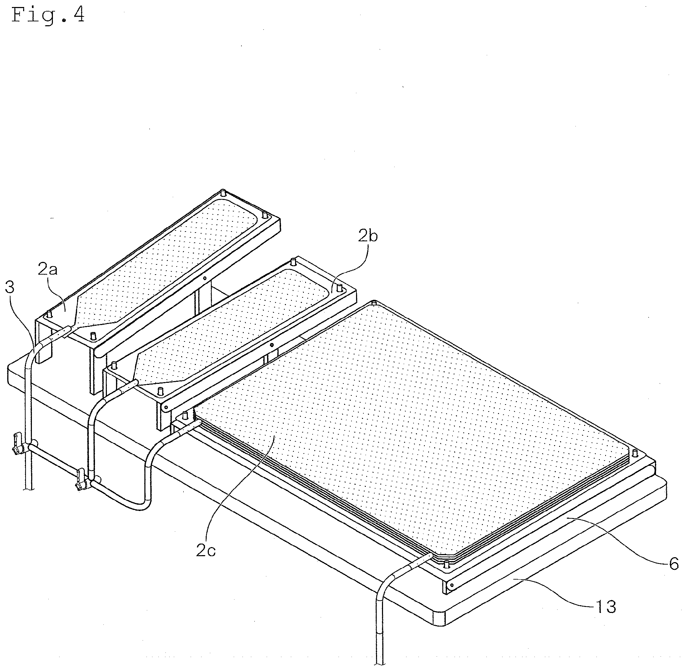

As shown in Fig. 3, the plurality of culture containers 2 are arranged such that a container connected nearer to the culture medium container 1 is positioned at a relatively higher position. That is, the primary culture container 2a is arranged at the highest position, the secondary culture container 2b is arranged at the second highest position, and the tertiary culture container 2c is positioned at the lowest position. By arranging the culture container 2 in this way, and by inclining the primary culture container 2a as shown in Fig. 4, it is possible to allow the liquid contents in the primary culture container 2a to be transferred by natural flow through the port below the primary culture container 2a and the transfer tube 3. Further, although not shown, by inclining the secondary culture container 2b similarly, it is possible to allow the liquid contents in the secondary culture container 2b to be transferred to the tertiary culture container 2c by natural flow through the port below the secondary culture container 2b and the transfer tube 3. In Fig. 3 and Fig. 4, the stirring means is not shown.

Although not shown, it is preferred that the culture medium container 1 be arranged at a position relatively lower than the culture container 2. For example, it can be arranged below a mounting table 13 on which the culture container is mounted. As a result, it becomes possible to prevent unintended flow of a culture medium to the culture system.

It is preferred that the plurality of the culture containers 2 and the culture medium container 1 be arranged in the same way as mentioned above also in the following embodiments.

Further, in the cell culture system according to the present embodiment, it is preferred that measuring means for measuring the weight of the base 6 on which the culture container 2 is mounted be provided. By monitoring, by using such measuring means, a decrease in weight of the base 6 on which the culture container 2 is mounted, if liquid is leaked from the culture container 2, it becomes possible to detect such leakage.

### (2) Primary culture step

By configuring the cell culture system in the above-mentioned way, cell culture is conducted in the primary culture container 2a at first. Further, in corresponding with proliferation or the like of cultured cells, a culture medium is supplied from the culture medium container 1 to the primary culture container 2a by means of a pump. At this time, it is preferred that the culture medium be transferred while increasing the temperature thereof by transferring at a speed slower than usual. The speed is preferably about 5% to 50% of the normal transfer speed, for example. Specifically, the transfer speed is preferably 5 mL/min to 50 mL/min, and more preferably 10 mL/min to 30 mL/min.

In this primary culture step, in order to allow the cells in the primary culture container 2a to be dispersed, it is preferable to apply vibration repeatedly to the primary culture container 2a by using the above-mentioned stirring means, thereby to stir the liquid contents in the primary culture container 2a. It is preferred that such stirring of the liquid contents be conducted similarly for other culture containers 2.

### (3) Cell transfer step 1

When the cell density in the primary culture container 2a becomes a prescribed cell density and the primary culture step has been completed, subsequently, cultured cells are transferred from the primary culture container 2a to the secondary culture container 2b.

At this time, as shown in Fig. 4, the primary culture container 2a is inclined such that the port for connecting it to the secondary culture container 2b is positioned at a lower side, the liquid contents (cell suspension) in the primary culture container 2a are flown to the secondary culture container 2b. As a result, cells cultured in the primary culture container 2a are transferred to the secondary culture container 2b. As mentioned above, the cells cultured in the primary culture container 2a are naturally flown to the secondary culture container 2b. When such flow is completed, normally, cells remain in the primary culture container 2a.

### (4) Culture medium addition step 1

Subsequently, the culture medium is added from the culture medium container 1 to the primary culture container 2a. At this time, as in the case where the culture medium is supplied in the primary culture step mentioned above, it is preferred that the culture medium be transferred while increasing the temperature thereof by transferring at a speed slower than usual.

By waiting for a prescribed period of time in a state where the culture medium is accommodated within the primary culture container 2a, the temperature of the culture medium is increased. This waiting time is preferably about 2 minutes to 20 minutes, for example, more preferably about 5 minutes to 10 minutes.

Subsequently, by the above-mentioned stirring means, the culture medium in the primary culture container 2a is stirred. Then, the culture medium is transferred from the primary culture container 2a to the secondary culture container 2b.

As a result, the culture medium of which the temperature is 15°C or less in the culture medium container 1 is transferred to the secondary culture container 2b after increasing the temperature thereof to 20°C or higher. Therefore, a low-temperature culture medium is not transferred to the secondary culture container 2b as it is, whereby lowering in proliferation efficiency of cells by the low-temperature culture medium can be prevented. Thereafter, the temperature of the culture medium is naturally increased to room temperature (for example, 37°C).

By transferring the culture medium to the secondary culture container 2b after being transferred to the primary culture container 2a, it is possible to transfer the cells remaining in the primary culture container 2a together with the culture medium to the secondary culture medium 2b, whereby the number of remaining cells in the cell culture can be reduced.

### (5) Secondary culture step

When the cultured cells and the culture medium are transferred to the secondary culture container 2b, culture of cells is conducted continuously in the secondary culture container 2b. Further, in corresponding with proliferation or the like of cultured cells, the culture medium can be supplied from the culture medium container 1. At this time, the supply can be conducted in the same way as in the culture medium addition step 1.

### (6) Cell transfer step 2

When the cell density in the secondary culture container 2b becomes a prescribed cell density and the secondary culture step has been completed, subsequently, cultured cells are transferred from the secondary culture container 2b to the tertiary culture container 2c.

At this time, the secondary culture container 2b is inclined such that the port for connecting it to the tertiary culture container 2c is positioned at a lower side, the liquid contents (cell suspension) in the secondary culture container 2b is flown to the tertiary culture container 2c. As a result, cells cultured in the secondary culture container 2b are transferred to the tertiary culture container 2c. As mentioned above, the cells cultured in the secondary culture container 2b are naturally flown to the tertiary culture container 2c. When such flow is completed, normally, cells remain in the secondary culture container 2b.

### (7) Culture medium addition step 2

Subsequently, the culture medium is added by flowing from the culture medium container 1 to the primary culture container 2a. At this time, as in the case of the culture medium addition step 1, the culture medium may be transferred at a speed slower than usual while increasing the temperature thereof. As in the case of the culture medium addition step 1, by waiting for a prescribed period of time in a state where the culture medium is accommodated within the primary culture container 2a, the temperature of the culture medium is increased.

Subsequently, after stirring the culture medium in the primary culture container 2a by means of a stirring means, the culture medium is transferred from the primary culture container 2a to the secondary culture container 2b. Then, in a state where the culture medium is accommodated within the secondary culture container 2b, the temperature of the culture medium is increased by waiting for a prescribed period of time. Subsequently, after stirring the culture medium in the secondary culture container 2b by means of a stirring means, the culture medium is transferred from the secondary culture container 2b to the tertiary culture container 2c.

At this time, since the culture medium is transferred to the tertiary culture container 2c after being transferred to the primary culture container 2a and the secondary culture container 2b, it is possible to transfer the cells remaining in the primary culture container 2a and the secondary culture container 2b together with the culture medium to the tertiary culture container 2c, whereby the number of remaining cells in the cell culture can be reduced.

### (8) Tertiary culture step

When the cultured cells and the culture medium are transferred to the tertiary culture container 2c, culture of cells is conducted continuously in the tertiary culture container 2c. Further, in corresponding with proliferation or the like of cultured cells, the culture medium can be supplied from the culture medium container 1. At this time, the supply can be conducted in the same way as in the culture medium addition step 2.

Meanwhile, the amount of the culture medium in the tertiary culture container 2c is large as compared with that in the primary culture container 2a or the secondary culture container 2b, and hence, effects caused by the cool culture medium are relatively small. Therefore, the transfer speed to the tertiary culture container 2c can be a normal transfer speed, e.g. about 100 mL/min, for example.

When the culture medium container 1 becomes empty when all of the culture media in the culture medium container 1 are supplied to the culture container 2, the empty culture medium container 1 can be used as a waste liquid container or a cell collection container.

### (9) Sampling step

When the number of cells or the density of cells in the tertiary culture container 2c become a prescribed value, sampling of the cells is conducted. This sampling step may be conducted during the amplification culture step.

The sampling of the cells is conducted such that, by stroking the sampling tube 2c-1, the liquid contents are sent to the sampling tube 2c-1 from the tertiary culture container 2c. The sampling tube 2c-1 is then thermally welded, followed by cutting. By doing so, sampling can be conducted aseptically while maintaining the dosed system of the cell culture system.

### (10) Concentration step

Subsequently, a supernatant of the liquid contents is flown from the tertiary culture container 2c by means of a pump to the culture medium container 1 as a waste liquid container. As a result, it becomes possible to increase the cell density in the liquid contents in the tertiary culture container 2c, whereby a cell suspension having a high concentration can be obtained. Then, cells can be collected from the tertiary culture container 2c that accommodates the thus-obtained cell suspension having a high concentration.

As mentioned above, by the cell culture method and the cell culture system according to the present embodiment, when the culture medium is supplied to the culture container, after the lapse of a prescribed period of time from the injection of the culture medium to a culture container in which cell culture has already been completed, the culture medium is transferred to the culture container. Therefore, not only the number of cells remaining in the culture container in which cell culture has already been completed can be reduced, but also the culture medium can be transferred to the culture container after the temperature thereof has been increased, whereby proliferation efficiency of cells can be further improved.

### (Second embodiment)

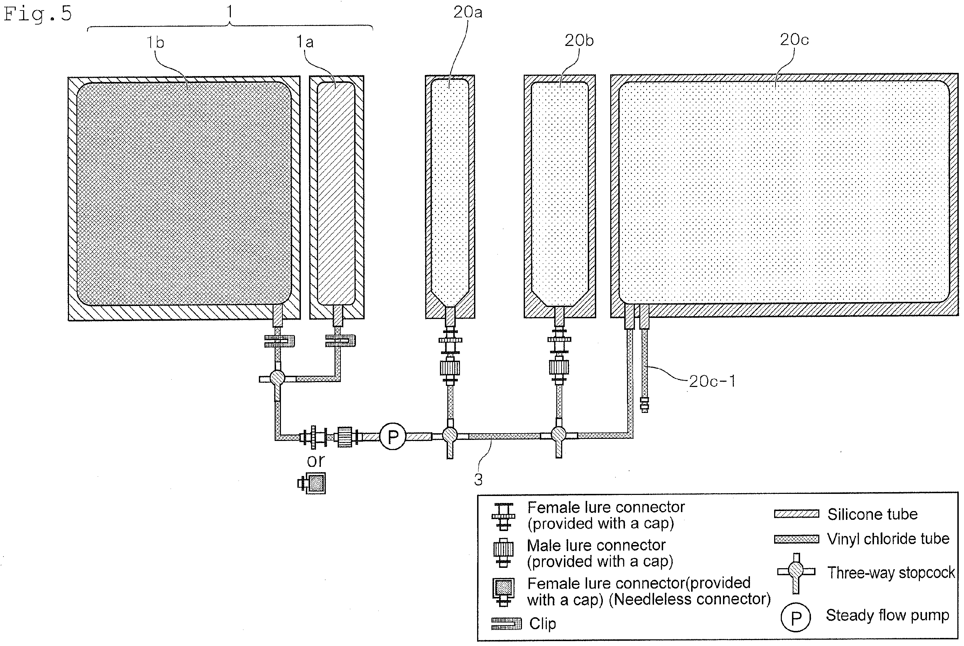

Subsequently, the cell culture method and the cell culture system according to the second embodiment of the present invention will be explained with reference to Fig. 5.

### [Cell culture system]

The cell culture system according to the present embodiment is used for culturing floating cells such as lymphocytes more efficiently. As shown in Fig. 5, it is configured that a plurality of culture medium containers 1 and a plurality of culture containers differing in function are connected by the transfer tube 3. Each of these plurality of culture containers is provided with one port for transferring contents with other containers. The culture medium container 1 and the plurality of culture containers 2 are connected by means of a tube.

Specifically, the cell culture system according to the present embodiment is provided with, as the culture medium supply container 1, a culture medium A supply container 1a and a culture medium B supply container 1b. In addition, it is provided with, as the plurality of culture containers, a curing and expansion culture container 20a (hereinafter, often referred to as a curing culture container or a curing container 20a), an activation culture container 20b (hereinafter, often referred to as an activation container 20b) and an amplification culture container 20c (hereinafter, often referred to as an amplification container 20c), and these containers are connected by means of the transfer tube 3.

The plurality of the culture containers is not limited to these three containers. For example, they may be two containers excluding the 20a; i.e. the activation container 20b and the amplification container 20c. The number of the culture containers may be four, five or more by using other culture containers or the like.

By the cell culture system according to the present embodiment, when cell culture is conducted in the activation container 20b after completion of the culture in the curing container 20a, prior to injection of the culture medium to the activation container 20b, the culture medium is injected to the curing container 20a first, followed by waiting for a prescribed period of time, and then the culture medium is transferred to the activation container 20b. As a result, the number of cells remaining in the curing container 20a can be reduced, and the temperature of the culture medium to be transferred to the activation container 20b can be increased. Further, when cell culture is conducted in the amplification container 20c, prior to injection of the culture medium to the amplification container 20c, the culture medium is injected to the curing container 20a and/or the activation container 20b, followed by waiting for a prescribed period of time, and then the culture medium is transferred to the amplification container 20c. As a result, not only the number of cells remaining in the curing container 20a and/or the activation container 20b can be reduced, but also the temperature of the culture medium to be transferred to the amplification container 20c can be further increased. As a result, proliferation efficiency of cells can be further improved.

### [Culture medium supply container]

The culture medium container 1 is a container for supplying a culture medium for culturing cells to a culture container, and stores a culture medium to be supplied to the curing container 20a, the activation container 20b and the amplification container 20c.

The culture medium container 1 is different from that of the first embodiment in that it is formed of the culture medium A supply container 1 a that supplies culture medium A to the curing container 20a and the activation container 20b and the culture medium B supply container 1 b that supplies culture medium B to the amplification container 20c, as shown in Fig. 5. The other points are the same as those in the first embodiment.

### [Curing and expansion culture container]

The curing container 20a is a container used to culture cells for recovering the function of damaged cells. That is, cells of which the functions have been lowered by being damaged (e.g. cells immediately after being taken out from a patient or cells that have been thawed after frozen storage) cannot be fully activated as they are. Therefore, such damaged cells cannot be cultured in a large amount. In order to proliferate these cells, a culture step (curing culture step) is conducted in which the cells are cultured while recovering them in the initial stage of cell culture so that the functions inherent to cells are recovered. Then, after the functions inherent to cells are recovered and proliferation of cells proceeds to some extent, the culture area is enlarged, and a culture step (expansion culture step) is conducted in which cell culture is continued until a prescribed cell density is attained.

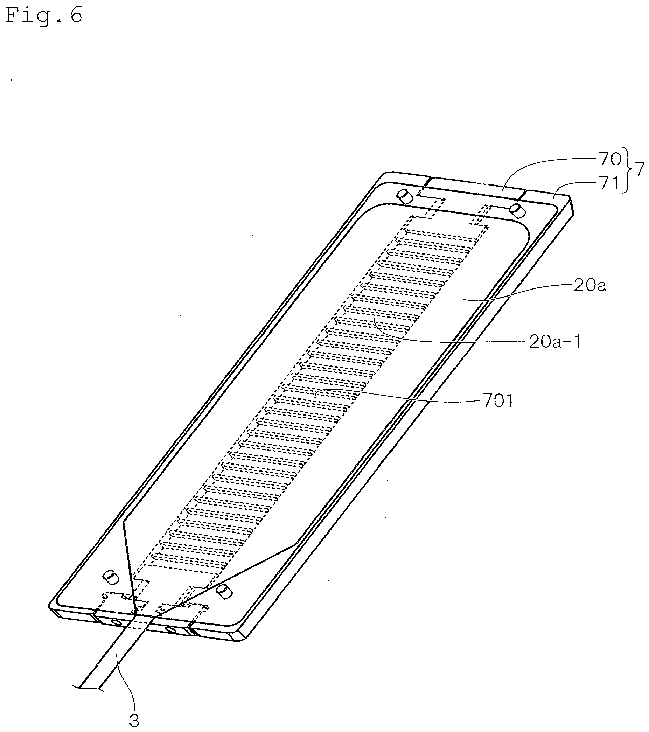

The curing container 20a is a culture container that is used in such curing culture step and expansion culture step, and, as shown in Fig. 6, is used together with a retaining tool 7 that retains the curing container 20a.

The curing container 20a is obtained by forming a soft packaging material into a bag-like shape. The soft packaging material means a packaging material that imparts flexibility and softness to a package.

Further, the curing container 20a has permeability to gas (permeability to oxygen and carbon dioxide) required for culturing cells. As a result, it is possible to conduct cell culture in a closed (sealed) system. It is preferable to use the curing container 20a under conditions of 37°C and 5% carbon dioxide concentration. The same can be applied to the activation container 20b and the amplification container 20c.

Further, part or all of the curing container 20a has transparency such that the content can be confirmed visually. It is preferred that the curing container 20a have low cytotoxicity, low elution properties, and suitability to radiation sterilization.

As the material of the curing container 20a that satisfies such conditions, a polyethylene-based resin is preferable. As the polyethylene-based resin, polyethylene, a copolymer of ethylene and α-olefin, a copolymer of ethylene and vinyl acetate, an ionomer obtained by using a copolymer of ethylene and acrylic acid or methacrylic acid and a metal ion, or the like can be given. Further, polyolefin, a styrene-based elastomer, a polyester-based thermoplastic elastomer, a silicone-based thermoplastic elastomer, a silicone resin or the like can also be used.

The shape of the curing container 20a is not particularly restricted. But, it can be rectangular, for example. One port is provided on one side of the short sides, and the transfer tube 3 is connected to this port Through this transfer tube 3, transfer of a cell suspension to the activation container 20b, addition of a culture medium from the culture medium A supply container 1 a to the curing container 20a and transfer of the culture medium from the curing container 20a to the activation container 20b are conducted.

It is preferred that the four sides of the curing container 20a be sealed by heat sealing, and that the accommodation part in the curing container 20a be gradually narrower towards a part at which the transfer tube 3 is attached. The curing container 20a may be an integrally-molded bag obtained by blow molding.

In the retaining tool 7 that retains the curing container 20a, a plurality of concave parts 701 are provided. It also has a bottom plate 70 that retains the middle part in the width direction of the curing container 20a and a side plate 71 that is provided along the edges of both sides of the bottom plate 70, retains the side peripheral part in the width direction of the curing container 20a, and can be turned around the lower end part thereof such that it can be switched from a vertically or obliquely elevated state to a horizontally collapsed state relative to the bottom plate 70. Fig. 6 shows a state in which the side plate 71 is horizontally collapsed relative to the bottom plate 70. The plurality of the concave parts 701 can be configured as through holes.

In order to retain the curing container 20a by attaching it to the retaining tool 7, on the side plate 71 of the retaining tool 7, a stopping element is vertically provided. At the same time, in the four corners of the curing container 20a, holes to be engaged with the stopping elements are provided. As a result, by engaging each of the stopping elements with each hole of the curing container 20a, the curing container 20a is attached to the retaining tool 7, and as shown in Fig. 6, the curing container 20a is held on an almost entire surface of the retaining tool 7 in which the side plate 71 and the bottom plate 70 are horizontally collapsed. In association with the turning of the side plate 71 from the elevated state to a state in which it is horizontally collapsed relative to the bottom plate 70, the four corners of the curing container 20a are pulled, whereby a tension is applied to the curing container 20a.

That is, when the side plate 71 is in an elevated state, the curing container 20a held on the bottom plate 70 is deformed by the weight of the content such that it partially falls down to the concave part 701, whereby a recess 20a-1 is formed in the bottom surface of the curing container 20a. Then, when the side plate 71 is turned such that it is collapsed horizontally relative to the bottom plate 70, the edge part in the width direction of the curing container 20a is collapsed together with the side plate 71, and in association with this movement, the four corners of the curing container 20a are pulled by the stopping element and tension is applied, whereby the deformation of the curing container 20a is removed. As a result, the recess 20a-1 formed in the bottom surface of the curing container 20a disappears.

According to the curing container 20a, when cell culture is started, if a cell suspension containing cells and a culture medium is accommodated in the curing container 20a, the cells are precipitated in the bottom surface of the curing container 20a. At this time, if the side plate 71 is kept in an elevated state, the precipitated cells are gathered to the recess 20a-1 formed in the bottom surface of the curing container 20a, whereby the cell density is increased.

As a result, cell culture can be conducted at the time of starting cell culture in a state that cell density is increased, whereby the functions inherent to the cells can be recovered more efficiently. Specifically, when floating cells such as lymphocytes are cultured, due to gathering of cells in the recess 20a-1 formed on the bottom surface of the curing container 20a, concentration of activators such as cytokines formed by cells is locally increased. Further, due to the interaction between cells, it has become possible to culture in a state where cell activity has been enhanced.

By removing deformation of the curing container 20a by applying tension thereto, it is possible to enlarge the culture area in the curing container 20a while removing the recess 20a-1 formed in the bottom surface of the curing container 20a. Therefore, it is possible to transfer from the curing culture step to the expansion culture step without changing containers, whereby damage on cells can be reduced and risk of contamination can be lowered.

By enlarging the culture area in the curing container 20a after proliferation of cells proceeds to some extent, cells can be cultured efficiently while keeping an appropriate cell density in the same container.

The curing container 20a in the present embodiment is one that can be particularly preferably used to recover cells of which the functions have been lowered by being damaged (e.g. cells immediately after being taken out from a patient or cells that have been thawed after frozen storage), and to culture such cells efficiently while recovering functions inherent to cells.

### [Activation culture container]

The activation container 20b is a container used to culture cells in order to activate cells. On the bottom surface of the activation container 20b, a substance that activates cells such as anti-CD3 antibodies are solid-phased. The cells accommodated within the activation container 20b are activated by bonding to the substance. The anti-CD3 antibodies are preferably used in order to activate lymphocytes.

As in the case of the curing container 20a, the activation container 20b is obtained by forming a soft packaging material into a bag, and part or all thereof has transparency such that the contents can be confirmed visually. As in the case of the curing container 20a, it is preferred that the activation container 20b have permeability to a gas (oxygen and carbon dioxide) required to culture cells and have low cytotoxicity, low elution properties, and suitability to radiation sterilization. As the material for the activation container 20b, the same as those of the curing container 20a can be used.

The shape of the activation container 20b is not particularly restricted. It can be rectangular, for example. One port is provided on one side of the shorter sides, and the transfer tube 3 is connected to this port. Through this transfer tube 3, transfer of a cell suspension to the amplification container 20c, addition of a culture medium from the culture medium A supply container 1 a or the curing container 20a to the activation container 20b and transfer of the culture medium from the activation container 20b to the amplification container 20c are conducted.

It is preferred that the four sides of the activation container 20b be sealed by heat sealing and that the accommodation part of the activation container 20b be gradually narrower towards a part where the transfer tube 3 is attached. The activation container 20b may be an integrally-molded bag obtained by blow molding.

It is preferred that the capacity of the activation container 20b be larger than the capacity of the curing container 20a. For example, it is preferable to use one having a capacity twice or more as large as the capacity of the curing container 20a.

### [Amplification culture container]

The amplification container 20c is a container for proliferating a large amount of cells.

The shape of the amplification container 20c is not particularly restricted. It can be rectangular, for example. One port for transferring contents with other containers is provided on one side thereof, and the transfer tube 3 is connected to this port. Through this transfer tube 3, transfer of a supernatant to the culture medium container 1 as the waste liquid container, transfer of a cell suspension to the culture medium container 1 as the collection container, and addition of a culture medium from the culture medium B supply container 1 b, the curing container 20a or the activation container 20b to the amplification container 20c are conducted. A port for connecting the sampling tube 20c-1 is also provided.

As for the amplification container 20c, it is preferable to use one having a large capacity in order to proliferate a large amount of cells. For example, it is preferable to use one having a capacity about 10 times as large as that of the activation container 20b.

The other points regarding the amplification container 20c in the cell culture system according to this embodiment are the same as those in the culture container 2 (tertiary culture container 2c) in the first embodiment The transfer tube 3 is the same as that of the first embodiment.

### [Cell culture method]

Next, the cell culture method according to the present embodiment will be explained in detail with reference to Fig. 5 and Fig. 6.

The cell culture method according to the present embodiment is a cell culture method wherein, when cells to be cultured are floating cells, as the plurality of culture containers,
a curing culture container used for cell culture for recovering functions of damaged cells,
an activation culture container used for activating cells, and
an amplification culture container used for proliferating cells are used,
a cell suspension containing cells and a culture medium is accommodated within the curing culture container, and the culture medium supply container, the curing culture container, the activation culture container and the amplification culture container are connected in this sequence,
after recovering the function of damaged cells in the curing culture container, a cell suspension containing cultured cells is transferred from the curing culture container to the activation culture container, a culture medium is supplied from the culture medium supply container to the curing culture container, and after the temperature of the culture medium is increased to a temperature that is equal to or higher than a prescribed value after the lapse of a prescribed period of time, the culture medium is supplied from the curing culture container to the activation culture container, and cell culture is conducted in the activation culture container, and
after activating cells in the activation culture container, a cell suspension containing activated cells is transferred from the activation culture container to the amplification culture container, a culture medium is supplied from the culture medium supply container to the curing culture container and/or the activation culture container, and after the temperature of the culture medium is increased to a temperature that is equal to or higher than a prescribed value after the lapse of a prescribed period of time, the culture medium is supplied from the curing culture container and/or the activation culture container to the amplification culture container, and cell culture is conducted in the amplification culture container to proliferate the cells.

Specifically, the cell culture method according to the present embodiment has (1) container connecting step; (2) curing culture step; (3) expansion culture step; (4) cell transfer step 1; (5) culture medium addition step 1; (6) activation culture step; (7) cell transfer step 2; (8) culture medium addition step 2; (9) amplification culture step; (10) sampling step; and (11) concentration step.

If two or more amplification containers 20c are used by connecting them each other, the steps (7) to (9) are repeatedly conducted for each amplification container 20c. The steps (10) and (11) can be omitted. In the present embodiment, since the steps (10) and (11) can be conducted in the same manner as in the first embodiment, an explanation will be omitted in the following.

### (1) Container connecting step

First, the culture medium container 1 and the plurality of culture containers 2 used in the cell culture method according to the present embodiment are connected as shown in Fig. 5, whereby a closed cell culture system is configured.

That is, the culture medium container 1, the curing container 20a, the activation container 20b and the amplification container 20c are connected in this sequence by the transfer tube 3.

At this time, the curing container 20a is connected in a state where a cell suspension containing cells to be cultured and a culture medium is accommodated. Further, among the culture medium containers 1, connection is attained in a state that, in the culture medium A supply container 1 a, culture medium A to be supplied to the curing container 20a and the activation container 20b is filled and, in the culture medium B supply container 1 b, culture medium B to be supplied to the amplification container 20c is filled. The activation container 20b and the amplification container 20c are connected in a state they are empty. On the transfer tube 3 between the culture medium container 1 and the curing container 20a, a pump is provided. By this pump, transfer or the like of a culture medium is conducted. On the transfer tube 3, three-way stopcocks are appropriately provided, and the liquid flow passage can be controlled by these.

### (2) Curing culture step

By configuring the cell culture system in this way, cell culture is conducted in the curing container 20a, and functions of damaged cells are recovered.

At this time, the retaining tool 7 that retains the curing container 20a is used in a state in which the side plate is elevated. The bottom surface of the curing container 20a is partially deformed by the weight of the content, and hence a recess is formed. Cells are gathered in this recess, whereby cell culture is conducted at a preferable cell density at the time of starting cell culture.

In corresponding with proliferation or the like of cultured cells, the culture medium A is supplied to the curing container 20a from the culture medium A supply container 1 a by means of a pump. At this time, as in the case of the first embodiment, it is preferred that the culture medium be transferred while increasing the temperature thereof by transferring at a speed slower than usual.

In order to allow the cells in the curing container 20a to be dispersed, it is preferable to stir the liquid contents in the curing container 20a by using the stirring means mentioned in the first embodiment It is preferred that such stirring of the liquid contents in the culture container be conducted similarly for the activation container 20b in the activation culture step mentioned later. It is preferred that such stirring be conducted for the amplification container 20c in the amplification culture step mentioned later. In the explanation regarding the activation culture step and the amplification culture step given later, an explanation on the stirring of the liquid contents in the culture container will be omitted.

### (3) Expansion culture step

When the functions of damaged cells are recovered and proliferation of cells proceeds to some extent, and the curing culture step is completed, an expansion culture step will be subsequently conducted.

In this step, as shown in Fig. 6, culture is conducted by enlarging the culture area in the curing container 20a by allowing the side plates of the retaining tool 7 to be horizontally collapsed.

At this time, the four comers of the curing container 20a held by the retaining tool 7 are pulled and tension is applied, whereby the deflection of the curing container 20a is removed, and as a result, the recesses formed in the bottom surface of the curing container 20a disappear. As a result, it becomes possible to allow cells to be dispersed on the entire bottom surface in the curing container 20a to conduct cell culture until a prescribed cell density is attained.

Also in this step, in corresponding with proliferation or the like of cultured cells, the culture medium A is supplied from the culture medium A supply container 1 a to the curing container 20a by means of a pump. Further, as in the case of the first embodiment, it is preferred that the culture medium be transferred while increasing the temperature thereof by transferring at a speed slower than usual.

### (4) Cell transfer step 1

When the cell density in the curing container 20a becomes a prescribed cell density and the curing culture step has been completed, subsequently, cultured cells are transferred from the curing container 20a to the activation container 20b.

At this time, the curing container 20a is inclined such that the port for connecting it to the activation container 20b is positioned at a lower side, and the liquid contents in the curing container 20a are flown to the activation container 20b. As a result, cells cultured in the curing container 20a are transferred to the activation container 20b. As mentioned above, the cells cultured in the curing container 20a are naturally flown to the activation container 20b. When such flow is completed, normally, cells remain in the curing container 20a.

### (5) Culture medium addition step 1

Subsequently, the culture medium is added from the culture medium A supply container 1a to the curing container 20a. At this time, as in the case of the first embodiment, it is preferred that the culture medium be transferred while increasing the temperature thereof by transferring at a speed slower than usual.

By waiting for a prescribed period of time in a state where the culture medium is accommodated within the curing container 20a, the temperature of the culture medium is increased as in the first embodiment.

Subsequently, by the above-mentioned stirring means, the liquid contents in the curing container 20a is stirred. Then, the culture medium is transferred from the curing container 20a to the activation container 20b.

By transferring the culture medium to the activation container 20b after transferring to the curing container 20a, it is possible to transfer the cells remaining in the curing container 20a together with the culture medium to the activation container 20b, whereby the number of remaining cells in the cell culture can be reduced.

### (6) Activation culture step

When transfer of cultured cells and a culture medium to the activation container 20b has been completed, activation of cells in the activation container 20b is conducted. When lymphocytes are cultured, anti-CD3 antibodies are solid-phased on the bottom surface in the activation container 20b, and the lymphocytes are activated by bonding to these anti-CD3 antibodies.

Also in the activation culture step, in corresponding with proliferation or the like of cultured cells, the culture medium can be supplied to the activation container 20b. At this time, it is preferred that addition of the culture medium be conducted as in the same manner in the culture medium addition step 1.

### (7) Cell transfer step 2

When the cells are fully activated in the activation container 20b and the activation culture step is completed, subsequently, cultured cells are transferred from the activation container 20b to the amplification container 20c.

At this time, by using the above-mentioned stirring means as the cell detaching means and by applying relatively strong vibration to the activation container 20b, it is possible to detach cells bonded to the antibodies that are solid-phased to the culture surface from the culture surface.

Then, the activation container 20b is inclined such that the port for connecting it to the amplification container 20c is positioned at a lower side, the liquid contents in the activation container 20b is flown to the amplification container 20c. As a result, cells cultured in the activation container 20b are transferred to the amplification container 20c. As mentioned above, the cells activated in the activation container 20b are naturally flown to the amplification container 20c. When such flow is completed, normally, cells remain in the activation container 20b.

### (8) Culture medium addition step 2

Subsequently, the culture medium is added from the culture medium B supply container 1 b to the curing container 20a. At this time, as in the case of the culture medium addition step 1, the culture medium may be transferred at a speed slower than usual while increasing the temperature thereof, and as in the case of the culture medium addition step 1, by waiting for a prescribed period of time in a state where the culture medium is accommodated within the curing container 20a, the temperature of the culture medium is increased.

Subsequently, by the stirring means, the liquid contents in the curing container 20a are stirred. Then, the culture medium is transferred from the curing container 20a to the activation container 20b, and by waiting for a prescribed period of time in a state where the culture medium is accommodated within the activation container 20b, the temperature of the culture medium is increased, and then the liquid contents in the activation container 20b are stirred by the stirring means, the culture medium is transferred from the activation container 20b to the amplification container 20c.

At this time, since the culture medium is transferred to the amplification container 20c after being transferred to the curing container 20a and the activation container 20b, it is possible to transfer the cells remaining in the curing container 20a and the activation container 20b together with the culture medium to the amplification container 20c, whereby the number of remaining cells in the cell culture can be reduced.

### (9) Amplification culture step

After completion of transfer of cultured cells and a culture medium to the amplification container 20c, in the amplification container 20c, a large amount of cells is proliferated. Proliferation of cells is conducted such that the number of cells or cell density in the amplification container 20c become a prescribed value.

In corresponding with proliferation or the like of cultured cells, the culture medium B is supplied from the culture medium B supply container 1 b to the amplification container 20c by means of a pump. At this time, addition of the culture medium can be conducted as in the case of the culture medium addition step 2.

When the culture medium B supply container 1b becomes empty when all of the culture media in the culture medium B supply container 1 b are supplied to the amplification container 20c, the culture medium B supply container 1 b that becomes empty can be used as a waste liquid container or a cell collection container.

As mentioned above, by the cell culture method and the cell culture system according to the present embodiment, when floating cells are cultured, it is possible to reduce the number of cells remaining in a culture container in which culture has been completed, and the culture medium is transferred to a culture container after increasing the temperature of the culture medium. As a result, it becomes possible to further improve the proliferation efficiency of cells.

### (Third embodiment)

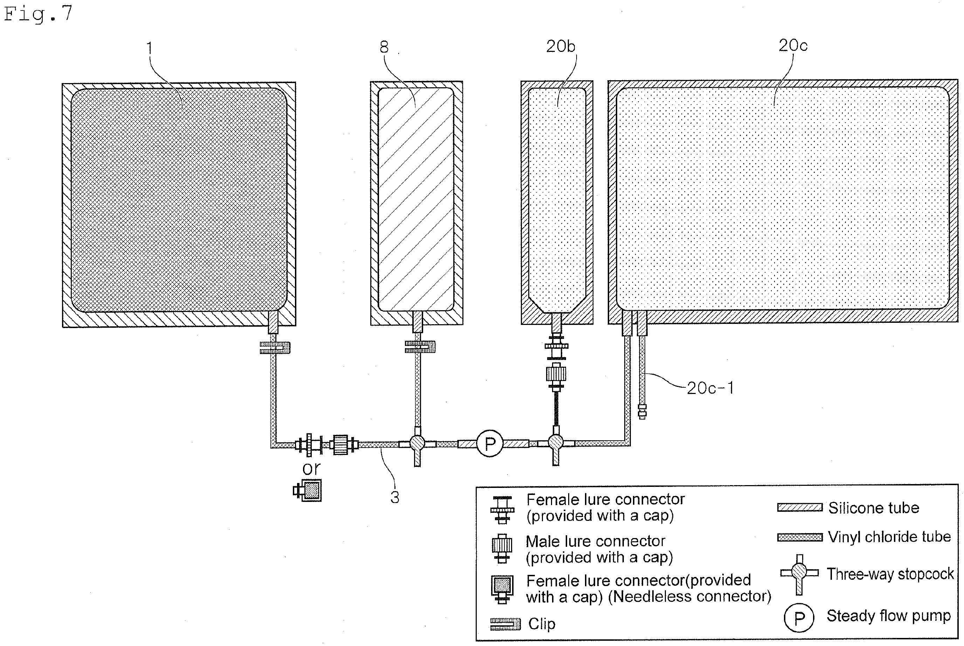

Next, the cell culture method and the cell culture system according to the third embodiment of the present invention will be explained with reference to Fig. 7.

The present embodiment is a modification example of the second embodiment. As shown in Fig. 7, the cell culture system according to the present embodiment has a configuration in which the culture medium container 1, the activation container 20b, the amplification container 20c and a collection container 8 are connected by the transfer tube 3.

That is, the cell culture system according to the present embodiment has a configuration in which the curing container 20a and the culture medium A supply container 1 a in the cell culture system in the second embodiment are omitted, and the collection container 8 is added. In this embodiment, a plurality of collection containers 8 may be provided.

In the cell culture method according to the present embodiment, no curing culture step is conducted, and cell culture is started from the activation culture step. By the collection container 8, it is possible to collect a supernatant of the amplification container 20c or collect a cell suspension from the amplification container 20c.

Other points are the same as those in the second embodiment.

By the cell culture method and the cell culture system according to the present embodiment, in a case where floating cells which are not required to be subjected to curing culture are cultured, etc., it is possible to reduce the number of cells remaining in a culture container in which cell culture has been completed, and to transfer a culture medium to a culture container after increasing the temperature of the culture medium. As a result, it is possible to further improve the proliferation efficiency of cells.

### (Fourth embodiment)

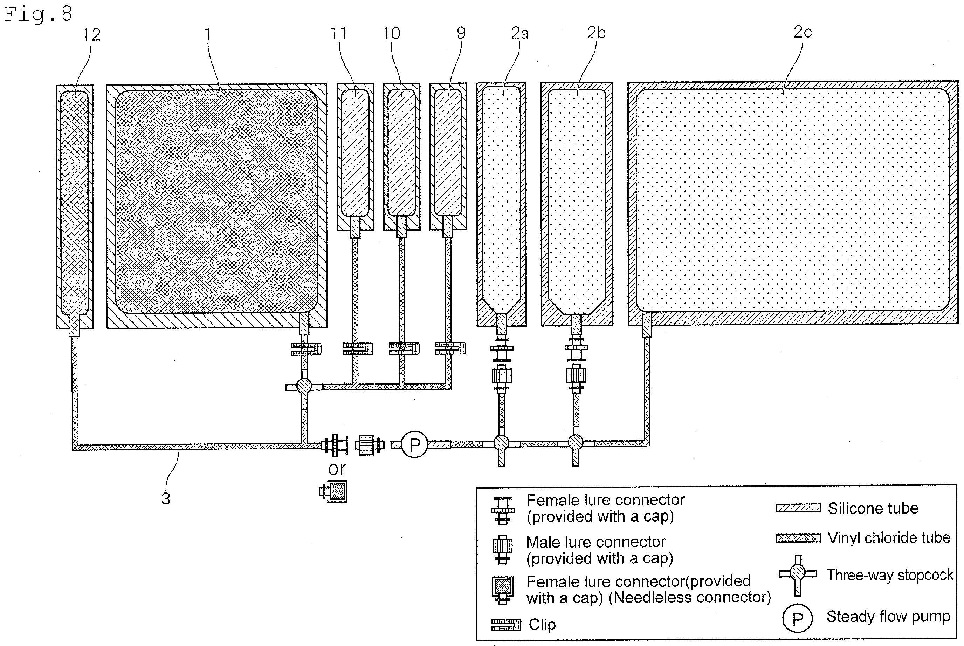

Subsequently, the cell culture method and the cell culture system according to the fourth embodiment of the present invention will be explained with reference to Fig. 8.

### [Cell culture system]

The cell culture system according to the present embodiment is a culture system for culturing adherent cells such as fibroblast cells more efficiently. As shown in Fig. 8, it has a configuration in which a waste liquid collection container 12 (hereinafter, often referred to as the waste liquid container 12), the culture medium container 1, a washing liquid supply container 9 (hereinafter, often referred to as the washing liquid container 9), an enzyme solution supply container 10 (hereinafter, often referred to as the enzyme container 10), an inhibitor solution supply container 11 (hereinafter, often referred to as the inhibitor container 11), and a plurality of culture containers 2 differing in culture area (primary culture container 2a, secondary culture container 2b, tertiary culture container 2c) are connected by means of the transfer tube 3.

For the same configurations as those in the first embodiment, an explanation will be omitted.

### [Washing liquid supply container]

The washing liquid container 9 is a container that supplies a washing liquid to the culture container 2. As shown in Fig. 8, it is connected between the culture medium container 1 and the primary culture container 2a by means of a tube.

A washing liquid is used to wash the culture medium remaining in the culture container 2 prior to the supply of an enzyme solution to the culture container 2.

As the washing liquid, one commonly used, such as physiological saline or PBS (phosphate buffered saline), can be used.

### [Enzyme solution supply container]

The enzyme container 10 is a container that supplies an enzyme solution for detaching cultured cells from the bottom surface of the culture container 2 to the culture container 2.

The enzyme solution is used to detach the cells from the bottom surface of the culture container 2 prior to the transfer of the cultured cells to a subsequent culture container 2 when cells have been proliferated over the entire culture surface of the culture container 2 and the cell culture in the culture container 2 has been completed. As such enzyme solution, one that can be normally used when adherent cells are detached from the bottom surface of the container can be used. For example, protease such as trypsin can be used.

### [Inhibitor solution supply container]

The inhibitor container 11 is a container that supplies to the culture container 2 an inhibitor solution for inhibiting activity of an enzyme in the enzyme solution.

The inhibitor solution is used to inhibit the activity of an enzyme that is used to detach=cells from the bottom surface of the culture container 2. As the inhibitor solution, one that can be normally used to inhibit activity of the enzyme can be used. For example, a protease inhibitor such as a trypsin inhibitor can be used. If the activity of an enzyme can be inhibited by a culture medium, the inhibitor container 11 in the cell culture system according to the present embodiment can be omitted.

### [Waste liquid collection container]

The waste liquid container 12 is a container that collects a washing liquid, an enzyme solution, an inhibitor solution, a culture medium or the like from the culture container 2, and these can be collected by means of a pump through the transfer tube 3.

### [Cell culture method]

Then, the cell culture method according to the present embodiment will be explained in detail with reference to Fig. 8.

The cell culture method according to the present embodiment is a cell culture method wherein, when cells to be cultured are adherent cells, as the plurality of culture containers, by using culture containers differing in culture area,
a cell suspension containing cells and a culture medium is accommodated within a first culture container having the smallest culture area among the plurality of culture containers, the plurality of culture containers are connected, in the ascending order from the smallest to the largest in terms of culture area, to the culture medium supply container that supplies a culture medium to the plurality of culture containers, and cell culture is started in the first culture container,
after completion of the culture in the first culture container, a cell suspension that contains cultured cells is transferred from the first culture container to a second culture container connected to the first culture container, and subsequently, a culture medium is supplied from the culture medium supply container to the first culture container, and after the lapse of a prescribed period of time, the culture medium is supplied from the first culture container to the second culture container to conduct cell culture in the second culture container, and
after completion of the culture in the second culture container, if there is a subsequent culture container connected to the culture container in which the culture has been completed, transfer of the cell suspension to the subsequent culture container, supply of a culture medium to the culture container in which the culture has been completed, supply of the culture medium from the culture container in which the culture has been completed to the subsequent culture container, and culture of cells in the subsequent culture container are conducted repeatedly until there is no subsequent culture container.

Specifically, the cell culture method according to the present embodiment comprises (1) container connection step; (2) primary culture step; (3) culture medium discharge step 1; (4) washing step 1; (5) cell detaching step 1; (6) enzyme activity inhibition step 1; (7) cell transfer step 1; (8) culture medium addition step 1; (9) enzyme etc. discharge step 1; (10) culture medium addition step 2; (11) secondary culture step; (12) culture medium discharge step 2; (13) washing step 2; (14) cell detaching step 2; (15) enzyme activity inhibiting step 2; (16) cell transfer step 2; (17) culture medium addition step 3; (18) enzyme etc. discharge step 2; (19) culture medium addition step 4; and (20) tertiary culture step. When four or more of the culture containers 2 are connected for use, as a step of the quaternary culture and the culture steps following the quaternary culture, the steps of (12) to (20) are further repeatedly conducted for each culture container 2.

### (1) Container connection step

First, the culture medium container 1, the plurality of culture containers 2, or the like used in the cell culture method according to the present embodiment are connected as shown in Fig. 8, whereby a closed cell culture system is configured.

Specifically, the waste liquid container 12, the culture medium container 1, the washing liquid container 9, the enzyme container 10, the inhibitor container 11, the primary culture container 2a, the secondary culture container 2b and the tertiary culture container 2c are connected by means of the transfer tube 3. Connection is conducted in a state that the culture medium container 1 is filled with a culture medium to be supplied to the culture container 2, the washing liquid container 9 is filled with a washing liquid, the enzyme container 10 is filled with an enzyme solution, and the inhibitor container 11 is filled with an inhibitor solution. The secondary culture container 2b, the tertiary culture container 2c and the waste liquid container 12 are connected in a state they are empty. On the transfer tube 3 between the culture medium container 1 and the culture container 2, a pump is provided. By means of the pump, transfer of a culture medium or the like is conducted. On the transfer tube 3, three-way stopcocks are appropriately provided, whereby the liquid flow passage can be controlled by these.

### (2) Primary culture step

By configuring the cell culture system in the above-mentioned way, cell culture is conducted in the primary culture container 2a having the smallest culture area at first. In the primary culture container 2a, primary culture of cells is conducted. Further, in corresponding with proliferation or the like of cultured cells, a culture medium is supplied from the culture medium container 1 to the primary culture container 2a by means of a pump. At this time, as in the case of the first embodiment, it is preferred that the culture medium be transferred while increasing the temperature thereof by transferring at a speed slower than usual.

### (3) Culture medium discharge step 1

When cells have been proliferated over the entire culture surface of the primary culture container 2a, the primary culture is completed. Then, the culture medium used for the culture is transferred from the primary culture container 2a to the waste liquid container 12 by means of a pump and discharged.

### (4) Washing step 1

Subsequently, the washing liquid is flown from the washing liquid container 9 to the primary culture container 2a, and by using the above-mentioned stirring means, the washing liquid in the primary culture container 2a is stirred and then discharged.

As a result, the culture medium remaining in the primary culture container 2a can be removed, and when an enzyme solution is flown to the primary culture container 2a in the subsequent cell detaching step 1, it becomes possible to prevent activity of an enzyme from being inhibited by a culture medium.

### (5) Cell detaching step 1

Subsequently, the enzyme solution is flown to the primary culture container 2a from the enzyme container 10, and cells adhering to the bottom surface of the primary culture container 2a are detached.

At this time, it is preferred that detachment of cells be promoted by stirring the liquid contents in the primary culture container 2a by using the above-mentioned stirring means. Promotion of detachment of cells by using the stirring means can be similarly applied to the secondary culture container 2b and other culture containers connected thereto.

### (6) Enzyme activity inhibiting step 1

Subsequently, an inhibitor solution is flown to the primary culture container 2a from the inhibitor container 11, thereby to inhibit the activity of the enzyme in the primary culture container 2a.

### (7) Cell transfer step 1

Subsequently, cells that have been detached are transferred from the primary culture container 2a to the secondary culture container 2b.

At this time, the primary culture container 2a is inclined such that the port for connecting it to the secondary culture container 2b is positioned at a lower side, the liquid contents containing detached cells can be naturally flown from the primary culture container 2a to the secondary culture container 2b.

When the detached cells are transferred from the secondary culture container 2b to the tertiary culture container 2c, similarly, transfer can be conducted by inclining the secondary culture container 2b such that the port for connecting it to the tertiary culture container 2c is positioned at a lower side.

### (8) Culture medium addition step 1

Subsequently, the culture medium is added from the culture medium container 1 to the primary culture container 2a. At this time, as in the case of the first embodiment, it is preferred that the culture medium be transferred while increasing the temperature thereof by transferring at a speed slower than usual. By waiting for a prescribed period of time in a state where the culture medium is accommodated within the primary culture container 2a, the temperature of the culture medium is increased.

Subsequently, after stirring the liquid contents in the primary culture container 2a by the stirring means, the culture medium is transferred from the primary culture container 2a to the secondary culture container 2b.

By transferring the culture medium to the secondary culture container 2b after transferring it to the primary culture container 2a, it becomes possible to supply the culture medium to the secondary culture container 2b after increasing the temperature thereof, as well as to reduce the number of cells remaining in the primary culture container 2a, whereby the proliferation efficiency of cells can be further improved.

### (9) Enzyme, etc. discharge step 1

When the cells are adhered to the bottom surface in the secondary culture container 2b, the enzyme solution, the inhibitor solution and the culture medium are discharged from the secondary culture container 2b to the waste liquid container 12 by means of a pump.

### (10) Culture medium addition step 2

Subsequently, a culture medium is again supplied to the secondary culture container 2b. At this time, as in the case of the culture medium addition step 1, it is preferred that the culture medium be transferred to the secondary culture container 2b after adding it by flowing from the culture medium container 1 to the primary culture container 2a to increase the temperature of the culture medium.

As a result, not only the culture medium can be supplied to the secondary culture container 2b after increasing the temperature thereof, but also the number of remaining cells in the primary culture container 2a can be further decreased, whereby proliferation efficiency of cells can be further improved.

### (11) Secondary culture step

By the above-mentioned steps, cell culture is conducted in the secondary culture container 2b having a larger culture area. Further, in corresponding with proliferation or the like of cultured cells, the culture medium is supplied from the culture medium container 1 to the secondary culture container 2b by means of a pump. At this time, it is preferred that addition of the culture medium be conducted in the same manner as in the culture medium addition step 1.

Subsequently, as the steps from (12) culture medium discharge step 2 to (19) culture medium addition step 4, the same steps as in the above-mentioned (3) culture medium discharge step 1 to (10) culture medium addition step 2 are conducted. At this time, it is preferred that addition of the culture medium be conducted in the same manner as in the culture medium addition step 2 in the first embodiment.

Subsequently, in (20) tertiary culture step, cell culture is conducted in the tertiary culture container 2c having a further larger culture area. In corresponding with proliferation or the like of cultured cells, the culture medium is supplied from the culture medium container 1 to the tertiary culture container 2c by means of a pump. At this time, addition of a culture medium may be conducted in the same manner as in the culture medium addition step 2 in the first embodiment.

When cells have been proliferated over the entire culture surface of the tertiary culture container 2c, the tertiary culture is completed, whereby it becomes possible to collect from the tertiary culture container 2c cultured cells that have been proliferated in a large amount.

As mentioned above, by the cell culture method and the cell culture system according to the present embodiment, when adherent cells are cultured, it is possible to reduce the number of cells remaining in a culture container in which culture has been completed, and at the same time, it is possible to transfer a culture medium to a culture container after increasing the temperature of the culture medium. As a result, proliferation efficiency of cells can be further improved.

Hereinabove, the present invention has been explained with reference to preferred embodiments. The present invention is not limited to the above-mentioned embodiments, and it is needless to say various modifications are possible within the scope of the present invention.

For example, in the above-mentioned second embodiment, an explanation has been made on an example in which the curing container 20a, the activation container 20b and the amplification container 20c are used. However, it is possible to make appropriate modifications. For example, the cell culture system according to the embodiment of the present invention is configured by using other plural containers differing in function or configurations in each embodiment are combined, or the like.

### Industrial Applicability

The present invention can be preferably used in regenerative medicine or preparation of antibody drugs where a large amount of cells is cultured in a closed system by using a culture container for cells.

Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.

The documents described in the specification are incorporated herein by reference in its entirety.

### Explanation of referential numerals

- 1.: Culture medium supply container (culture medium container)
- 1a.: Culture medium A supply container
- 1b.: Culture medium B supply container
- 2.: Culture container
- 2a.: Primary culture container
- 20a.: Curing and expansion culture container (curing culture container, curing container)
- 20a-1.: Recess
- 2b.: Secondary culture container
- 20b.: Activation culture container (activation container)
- 2c.: Tertiary culture container
- 2c-1.: Sampling tube
- 20c.: Amplification culture container (amplification container)
- 20c-1.: Sampling tube
- 3.: Transfer tube
- 4.: Holding part
- 41.: Holding plate
- 42.: Turning shaft
- 5.: Plate cam
- 51.: First cam surface
- 52.: Second cam surface
- 53.: Driving shaft
- 6.: Base
- 7.: Retaining tool
- 70.: Bottom plate
- 701.: Concave part
- 71.: Side plate
- 8.: Collection container
- 9.: Washing liquid supply container (washing liquid container)
- 10.: Enzyme solution supply container (enzyme container)
- 11.: Inhibitor solution supply container (inhibitor container)
- 12.: Waste liquid collection container (waste liquid container)
- 13.: Mounting table

## Claims

1. A cell culture method in which cells are cultured by using a plurality of culture containers, wherein
each of the plurality of culture containers is provided with one port for transferring contents with other containers,
a cell suspension containing cells and a culture medium is accommodated within a first culture container among the plurality of culture containers,
a culture medium supply container that supplies a culture medium to the plurality of culture containers and the first culture container are connected by means of a tube, and other culture containers among the plurality of culture containers are connected in sequence to the first culture container by means of a tube,
cell culture is started in the first culture container,
after completion of the culture in the first culture container, a cell suspension that contains cultured cells is transferred from the first culture container to a second culture container connected to the first culture container, and subsequently, a culture medium is supplied from the culture medium supply container to the first culture container, and after the lapse of a prescribed period of time, the culture medium is supplied from the first culture container to the second culture container to conduct cell culture in the second culture container, and
after completion of the culture in the second culture container, if there is a subsequent culture container connected to the culture container in which the culture has been completed, transfer of the cell suspension to the subsequent culture container, supply of a culture medium to the culture container in which the culture has been completed, supply of the culture medium from the culture container in which the culture has been completed to the subsequent culture container, and culture of cells in the subsequent culture container are conducted repeatedly until there is no subsequent culture container

2. The cell culture method according to claim 1, wherein
when cells to be cultured are floating cells, as the plurality of culture containers,
a curing culture container used for cell culture for recovering functions of damaged cells,
an activation culture container used for activating cells, and
an amplification culture container used for proliferating cells
are used,
a cell suspension containing cells and a culture medium is accommodated within the curing culture container, and the culture medium supply container, the curing culture container, the activation culture container and the amplification culture container are connected in this sequence,
after recovering the function of damaged cells in the curing culture container, a cell suspension containing cultured cells is transferred from the curing culture container to the activation culture container, a culture medium is supplied from the culture medium supply container to the curing culture container, and after the temperature of the culture medium is increased to a temperature that is equal to or higher than a prescribed value after the lapse of a prescribed period of time, the culture medium is supplied from the curing culture container to the activation culture container, and cell culture is conducted in the activation culture container, and
after activating cells in the activation culture container, a cell suspension containing activated cells is supplied from the activation culture container to the amplification culture container, a culture medium is supplied from the culture medium supply container to the curing culture container and/or the activation culture container, and after the temperature of the culture medium is increased to a temperature that is equal to or higher than a prescribed value after the lapse of a prescribed period of time, the culture medium is supplied from the curing culture container and/or the activation culture container to the amplification culture container, and cell culture is conducted in the amplification culture container to proliferate the cells.

3. The cell culture method according to claim 2, wherein, after activating cells in the activation culture container, a cell suspension containing activated cells is supplied from the activation culture container to the amplification culture container, a culture medium is supplied from the culture medium supply container to the curing culture container, and after the lapse of a prescribed period of time, the culture medium is supplied from the curing culture container to the activation culture container, and after the lapse of a prescribed period of time, the culture medium is supplied from the activation culture container to the amplification culture container, and cell culture is conducted in the amplification culture container to proliferate the cells.

4. The cell culture method according to claim 1, wherein, when cells to be cultured are floating cells, as the plurality of culture containers,
an activation culture container used for activating cells and
an amplification culture container used for proliferating cells
are used,
a cell suspension containing cells and a culture medium is accommodated within the activation culture container, and the culture medium supply container, the activation culture container and the amplification culture container are connected in this sequence, and
after activating cells in the activation culture container, a cell suspension containing activated cells is supplied from the activation culture container to the amplification culture container, a culture medium is supplied from the culture medium supply container to the activation culture container, and after the temperature of the culture medium is increased to a temperature that is equal to or higher than a prescribed value after the lapse of a prescribed period of time, the culture medium is supplied from the activation culture container to the amplification culture container, and cell culture is conducted in the amplification culture container to proliferate the cells.

5. The cell culture method according to claim 1, wherein, when cells to be cultured are adherent cells, as the plurality of culture containers, by using culture containers differing in culture area,
a cell suspension containing cells and a culture medium is accommodated within a first culture container having the smallest culture area among the plurality of culture containers, the plurality of culture containers are connected, in the ascending order from the smallest to the largest in terms of culture area, to the culture medium supply container that supplies a culture medium to the plurality of culture containers, and cell culture is started in the first culture container.

6. The cell culture method according to claim 5, wherein
an enzyme solution supply container that supplies an enzyme solution for detaching cultured cells from the bottom surface of the culture container to the culture container is connected between the culture medium supply container and the first culture container by means of a tube, and
the enzyme solution is flown from the enzyme solution supply container to the culture container, cultured cells are detached from the bottom surface of the culture container, and transfer of the cell suspension containing cultured cells is conducted.

7. The cell culture method according to any one of claims 1 to 6, wherein the first culture container connected to the culture medium supply container, the second culture container connected to the first culture container, and if there are other culture containers connected, these other containers, are arranged such that a culture container connected nearer to the culture medium supply container is positioned at a relatively higher position, and
each culture container is inclined to conduct transfer of a cell suspension from the culture container to the subsequently connected culture container.

8. The cell culture method according to any one of claims 1 to 7, wherein the plurality of culture containers are separately or collectively held in a holding part having one or two or more flat plate-shaped holding plates in which a turning shaft provided on one end thereof is pivotably supported on a base;
the holding plate is turned upward around the turning shaft, thereby allowing the plurality of culture containers to be moved upward to a position higher than the initial position while being inclined separately or simultaneously, and
the holding plate is turned downward around the turning shaft and the holding plate contacts with the base when the plurality of culture containers are returned to the initial position separately or simultaneously, whereby vibration applied to the plurality of culture containers is generated.

9. A cell culture system that is provided with a plurality of culture containers for culturing floating cells, comprising:
a culture medium supply container that supplies a culture medium to the plurality of culture containers, and
as the plurality of culture containers,
a curing culture container used for cell culture for recovering functions of damaged cells,
an activation culture container used for cell culture for activating cells, and
one or two or more amplification culture containers used for cell culture for proliferating cells, wherein
each of the curing culture container, the activation culture container and the amplification culture container is provided with one port for transferring contents with other containers, and
the culture medium supply container, the curing culture container, the activation culture container and the amplification culture container are connected in this sequence by means of a tube.

10. A cell culture system that is provided with a plurality of culture containers for culturing adherent cells, comprising:
a culture medium supply container that supplies a culture medium to the plurality of culture containers, and
culture containers differing in culture area as the plurality of culture containers, wherein
each of the plurality of culture containers is provided with one port for transferring contents with other containers, and
the plurality of culture containers are connected, in the ascending order from the smallest to the largest in terms of culture area, by means of a tube to the culture medium supply container.

11. The cell culture system according to claim 9 or 10 that is provided with a holding part having one or two or more flat plate-shaped holding plates in which a turning shaft provided on one end thereof is pivotably supported on a base, and a cam mechanism in which the holding plate is used as a follower, wherein
the plurality of culture containers are held in the holding part separately or collectively, and
the holding plate is turned upward and downward repeatedly around the turning shaft by the cam mechanism, and vibration generated when the holding plate is turned downward and contacts with the base is applied separately or simultaneously to the plurality of culture containers, whereby a liquid contents in the plurality of culture containers is stirred.

## Amended claims

### Amended claims under Art. 19.1 PCT

1. A cell culture method in which cells are cultured by using a plurality of culture containers, wherein
each of the plurality of culture containers is provided with one port for transferring contents with other containers,
a cell suspension containing cells and a culture medium is accommodated within a first culture container among the plurality of culture containers,
a culture medium supply container that supplies a culture medium to the plurality of culture containers and the first culture container are connected by means of a tube, and other culture containers among the plurality of culture containers are connected in sequence to the first culture container by means of a tube,
cell culture is started in the first culture container,
after completion of the culture in the first culture container, a cell suspension that contains cultured cells is transferred from the first culture container to a second culture container connected to the first culture container, and subsequently, a culture medium is supplied from the culture medium supply container to the first culture container, and after the lapse of a prescribed period of time, the culture medium is supplied from the first culture container to the second culture container to conduct cell culture in the second culture container, and
after completion of the culture in the second culture container, if there is a subsequent culture container connected to the culture container in which the culture has been completed, transfer of the cell suspension to the subsequent culture container, supply of a culture medium to the culture container in which the culture has been completed, supply of the culture medium from the culture container in which the culture has been completed to the subsequent culture container, and culture of cells in the subsequent culture container are conducted repeatedly until there is no subsequent culture container.

2. The cell culture method according to claim 1, wherein
when cells to be cultured are floating cells, as the plurality of culture containers,
a curing culture container used for cell culture for recovering functions of damaged cells,
an activation culture container used for activating cells, and
an amplification culture container used for proliferating cells are used,
a cell suspension containing cells and a culture medium is accommodated within the curing culture container, and the culture medium supply container, the curing culture container, the activation culture container and the amplification culture container are connected in this sequence,
after recovering the function of damaged cells in the curing culture container, a cell suspension containing cultured cells is transferred from the curing culture container to the activation culture container, a culture medium is supplied from the culture medium supply container to the curing culture container, and after the temperature of the culture medium is increased to a temperature that is equal to or higher than a prescribed value after the lapse of a prescribed period of time, the culture medium is supplied from the curing culture container to the activation culture container, and cell culture is conducted in the activation culture container, and
after activating cells in the activation culture container, a cell suspension containing activated cells is supplied from the activation culture container to the amplification culture container, a culture medium is supplied from the culture medium supply container to the curing culture container and/or the activation culture container, and after the temperature of the culture medium is increased to a temperature that is equal to or higher than a prescribed value after the lapse of a prescribed period of time, the culture medium is supplied from the curing culture container and/or the activation culture container to the amplification culture container, and cell culture is conducted in the amplification culture container to proliferate the cells.

3. The cell culture method according to claim 2, wherein, after activating cells in the activation culture container, a cell suspension containing activated cells is supplied from the activation culture container to the amplification culture container, a culture medium is supplied from the culture medium supply container to the curing culture container, and after the lapse of a prescribed period of time, the culture medium is supplied from the curing culture container to the activation culture container, and after the lapse of a prescribed period of time, the culture medium is supplied from the activation culture container to the amplification culture container, and cell culture is conducted in the amplification culture container to proliferate the cells.

4. The cell culture method according to claim 1, wherein, when cells to be cultured are floating cells, as the plurality of culture containers,
an activation culture container used for activating cells and
an amplification culture container used for proliferating cells are used,
a cell suspension containing cells and a culture medium is accommodated within the activation culture container, and the culture medium supply container, the activation culture container and the amplification culture container are connected in this sequence, and
after activating cells in the activation culture container, a cell suspension containing activated cells is supplied from the activation culture container to the amplification culture container, a culture medium is supplied from the culture medium supply container to the activation culture container, and after the temperature of the culture medium is increased to a temperature that is equal to or higher than a prescribed value after the lapse of a prescribed period of time, the culture medium is supplied from the activation culture container to the amplification culture container, and cell culture is conducted in the amplification culture container to proliferate the cells.

5. The cell culture method according to claim 1, wherein, when cells to be cultured are adherent cells, as the plurality of culture containers, by using culture containers differing in culture area,
a cell suspension containing cells and a culture medium is accommodated within a first culture container having the smallest culture area among the plurality of culture containers, the plurality of culture containers are connected, in the ascending order from the smallest to the largest in terms of culture area, to the culture medium supply container that supplies a culture medium to the plurality of culture containers, and cell culture is started in the first culture container.

6. The cell culture method according to claim 5, wherein
an enzyme solution supply container that supplies an enzyme solution for detaching cultured cells from the bottom surface of the culture container to the culture container is connected between the culture medium supply container and the first culture container by means of a tube, and
the enzyme solution is flown from the enzyme solution supply container to the culture container, cultured cells are detached from the bottom surface of the culture container, and transfer of the cell suspension containing cultured cells is conducted.

7. The cell culture method according to any one of claims 1 to 6, wherein the first culture container connected to the culture medium supply container, the second culture container connected to the first culture container, and if there are other culture containers connected, these other containers, are arranged such that a culture container connected nearer to the culture medium supply container is positioned at a relatively higher position, and
each culture container is inclined to conduct transfer of a cell suspension from the culture container to the subsequently connected culture container.

8. The cell culture method according to any one of claims 1 to 7, wherein the plurality of culture containers are separately or collectively held in a holding part having one or two or more flat plate-shaped holding plates in which a turning shaft provided on one end thereof is pivotably supported on a base;
the holding plate is turned upward around the turning shaft, thereby allowing the plurality of culture containers to be moved upward to a position higher than the initial position while being inclined separately or simultaneously, and
the holding plate is turned downward around the turning shaft and the holding plate contacts with the base when the plurality of culture containers are returned to the initial position separately or simultaneously, whereby vibration applied to the plurality of culture containers is generated.

9. A cell culture system that is provided with a plurality of culture containers for culturing floating cells, comprising:
a culture medium supply container that supplies a culture medium to the plurality of culture containers, and
as the plurality of culture containers,
a curing culture container used for cell culture for recovering functions of damaged cells,
an activation culture container used for cell culture for activating cells, and
one or two or more amplification culture containers used for cell culture for proliferating cells, wherein
each of the curing culture container, the activation culture container and the amplification culture container is provided with one port for transferring contents with other containers, and
the culture medium supply container, the curing culture container, the activation culture container and the amplification culture container are connected in this sequence by means of a tube.

10. A cell culture system that is provided with a plurality of culture containers for culturing adherent cells, comprising:
a culture medium supply container that supplies a culture medium to the plurality of culture containers, and
culture containers differing in culture area as the plurality of culture containers, wherein
each of the plurality of culture containers is provided with one port for transferring contents with other containers, and
the plurality of culture containers are connected, in the ascending order from the smallest to the largest in terms of culture area, by means of a tube to the culture medium supply container.

11. The cell culture system according to claim 9 or 10 that is provided with a holding part having one or two or more flat plate-shaped holding plates in which a turning shaft provided on one end thereof is pivotably supported on a base, and a cam mechanism in which the holding plate is used as a follower, wherein
the plurality of culture containers are held in the holding part separately or collectively, and
the holding plate is turned upward and downward repeatedly around the turning shaft by the cam mechanism, and vibration generated when the holding plate is turned downward and contacts with the base is applied separately or simultaneously to the plurality of culture containers, whereby a liquid contents in the plurality of culture containers is stirred.

12. (added) The cell culture method according to any one of claims 1 to 8, wherein at least any of the plurality of culture containers is provided with a port for sampling, a sampling tube is connected to the port for sampling, and an end opposite to the port for sampling of the sampling tube is sealed,
the sampling tube is stroked to allow the sampling tube to be filled with a cell suspension from the culture container to which the sampling tube is connected, and
the sampling tube is cut after being thermally welded.

13. (added) The cell culture system according to any one of claims 9 to 11, wherein at least any of the plurality of culture containers is provided with a port for sampling, and a sampling tube is connected to the port for sampling, and an end opposite to the port for sampling of the sampling tube is sealed.
Statement
The claims 12 and 13 are based on the paragraph [0031] of the description and Fig 1 of the drawings as filed.
